# EUROPEAN PATENT SPECIFICATION

(11) **EP 3 010 519 B1**
(45) Date of publication and mention of the grant of the patent: **07.04.2021**
(21) Application number: 14736238.8
(22) Date of filing: 13.06.2014
(51) Int. Cl.: A61K 35/76, A61P 35/00

(54) **ONCOLYTIC ADENOVIRUS FOR USE IN A TREATMENT OF BRAIN CANCER**
ONKOLYTISCHEN ADENOVIREN ZUR VERWENDUNG IN DER BEHANDLUNG VON HIRNKREBS
ADÉNOVIRUS ONCOLYTIQUE POUR L'UTILISATION DANS LE TRAITEMENT DU CANCER DU CERVEAU

(30) Priority: 18.06.2013 US 201361836230 P
(43) Date of publication of application: 27.04.2016
(73) Proprietor: DNAtrix, Inc., Houston, TX 77046 (US); Board of Regents, The University of Texas System, Austin, TX 78701 (US)
(72) Inventor: FUEYO, Juan, Houston, TX 77030 (US); MANZANO-GOMEZ, Candeleria, Houston, TX 77046 (US); CONRAD, Charles, deceased (US); LANG, Fred, Houston, TX 77046 (US); YUNG, W.k., Alfred, Houston, TX 77046 (US); TUFARO, Frank, Houston, TX 77046 (US)
(74) Representative: Potter Clarkson
(86) International application number: PCT/US2014/042375
(87) International publication number: WO 2014/204814

(56) References cited:
- WO-A1-2013/076374
- US-A1- 2003 138 405
- Marta M Alonso: "Can Oncolytic Adenovirus Be Implemented as Therapeutic Strategies for DIPGs? DIPG EUROPEAN MEETING Barcelona 23-24 February 2012", , 23 February 2012 (2012-02-23), XP055142310, Retrieved from the Internet: URL:http://www.cristianriverafoundation.or g/media/DIPGworkshopsmedia/202 - SPAIN - Oncolytic Adenovirus (Alonso).pdf [retrieved on 2014-09-24]
- MARTA M ALONSO ET AL: "Delta-24-RGD in Combination With RAD001 Induces Enhanced Anti-glioma Effect via Autophagic Cell Death", MOLECULAR THERAPY, vol. 16, no. 3, 1 March 2008 (2008-03-01), pages 487-493, XP055142302, ISSN: 1525-0016, DOI: 10.1038/sj.mt.6300400
- NOBUSADA SHIMOURA ET AL: "HIGHLY AUGMENTED CYTOPATHIC EFFECT OF A FIBER-MUTANT E1B-DEFECTIVE ADENOVIRUS FOR GENE THERAPY OF GLIOMAS", CANCER RESEARCH, AMERICAN ASSOCIATION FOR CANCER RESEARCH, US, vol. 59, no. 14, 1 July 1999 (1999-07-01), pages 3411-3416, XP002927072, ISSN: 0008-5472
- Hong Jiang ET AL: "Oncolytic Adenovirus: Preclinical and Clinical Studies in Patients with Human Malignant Gliomas", Current Gene Therapy, vol. 9, no. 5, 1 October 2009 (2009-10-01) , pages 422-427, XP055358049, NL ISSN: 1566-5232, DOI: 10.2174/156652309789753356

## Description

### BACKGROUND OF THE INVENTION

### A. Field of the Invention

The invention generally relates to the field of medicine and oncology. More particularly, it concerns compositions and methods of treating gliomas in a patient using oncolytic adenoviruses.

### B. DESCRIPTION of Related Art

The development of cancer is understood as the culmination of complex, multistep biological processes, occurring through the accumulation of genetic alterations. Many if not all of these alterations involve specific cellular growth-controlling genes. These genes typically fall into two categories: proto-oncogenes and tumor suppressor genes. Mutations in genes of both classes generally confer a growth advantage on the cell containing the altered genetic material.

The function of tumor suppressor genes, as opposed to proto-oncogenes, is to antagonize cellular proliferation. When a tumor suppressor gene is inactivated, for example by point mutation or deletion, the cell's regulatory machinery for controlling growth is upset. Mutations and/or loss of function in the retinoblastoma tumor suppressor gene have been associated with tumor formation. In some instances brain tumors are metastases to the brain from a primary tumor outside of the central nervous system (CNS). Brain tumors derived from metastases are typically more common than primary tumors of the brain. The most common primary tumors that metastasize to the brain are lung, breast, melanoma, and kidney. These brain metastases are usually in multiple sites, but solitary metastases may also occur.

Gene therapy is a promising treatment for brain tumors including gliomas because conventional therapies typically fail and are toxic. In addition, the identification of genetic abnormalities contributing to malignancies is providing crucial molecular genetic information to aid in the design of gene therapies. Genetic abnormalities indicated in the progression of tumors include the inactivation of tumor suppressor genes and the overexpression of numerous growth factors and oncogenes. Tumor treatment may be accomplished by supplying a polynucleotide encoding a therapeutic polypeptide or other therapeutic that target the mutations and resultant aberrant physiologies of tumors. It is these mutations and aberrant physiology that distinguishes tumor cells from normal cells. A tumor-selective virus would be a promising tool for gene therapy. Recent advances in the knowledge of how viruses replicate have been used to design tumor-selective oncolytic viruses. In gliomas, three kinds of viruses have been shown to be useful in animal models: reoviruses that can replicate selectively in tumors with an activated ras pathway (Coffey *et al*., 1998); genetically altered herpes simplex viruses (Martuza *et al*., 1991; Mineta *et al*., 1995; Andreanski *et al*., 1997), including those that can be activated by the different expression of proteins in normal and cancer cells (Chase *et al*., 1998); and mutant adenoviruses that are unable to express the E1B55kDa protein and are used to treat *p53*-mutant tumors (Bischof *et al*., 1996; Heise *et al*., 1997; Freytag *et al*., 1998; Kirn *et al*., 1998). Taken together, these reports confirm the relevance of oncolytic viruses as anti-cancer agents. In all three systems, the goal is the intratumoral spread of the virus and the ability to selectively kill cancer cells. Genetically modified adenoviruses that target cellular pathways at key points have both potent and selective anti-cancer effects in gliomas.

Targeting the Rb pathway has noted relevance for the treatment of gliomas because abnormalities of the p16/Rb/E2F pathway are present in most gliomas (Fueyo *et al*., 1998a; Gomez-Manzano *et al*., 1998). Targeting this pathway by replacement of lost tumor suppressor activity through the transfer of *p16* and Rb genes has produced cytostatic effects (Fueyo *et al*., 1998a; Gomez-Manzano *et al*., 1998). Transfer of *E2F-1* resulted in powerful anti-cancer effect since the exogenous wild-type E2F-1 induced apoptosis and inhibited tumor growth *in vivo* (Fueyo *et al*., 1998b). However, treating human glioma tumors with existing adenovirus constructs realistically cannot affect significant portions of the tumor, mainly because replication-deficient adenoviral vectors are unable to replicate and infect other cells, thus transferring the exogenous nucleic acid to sufficient numbers of cancer cells (Puumalainen *et al*., 1998). Although targeting the p16/Rb/E2F pathway produces an anti-cancer effect *in vitro,* this imperfection of the vector system limits the therapeutic effect of the gene *in vivo.*

There is a continued need for additional treatments for cancer, particularly brain tumors, including the creation of additional oncolytic viruses that are capable of cell-specific replication. Additional treatments include an adenovirus with therapeutic capabilities or with an ability to be tracked *in vivo.*

### SUMMARY OF THE INVENTION

Thus, in accordance with the present invention, there is provided an oncolytic adenovirus for use in one or more of: (i) a method for achieving greater than 25% reduction in tumor burden when treating a glioma in a human patient without producing of an adverse event resulting from said oncolytic adenovirus that is sufficient to cause termination of said treatment; (ii) a method for achieving a six-month progression-free survival when treating a glioma in a human patient without producing of an adverse event resulting from said oncolytic adenovirus that is sufficient to cause termination of said treatment; and (iii) a method for achieving tumor necrosis when treating a glioma in a human patient without producing of an adverse event resulting from said oncolytic adenovirus that is sufficient to cause termination of said treatment, wherein the method comprises contacting the glioma, in the human patient identified as having the glioma, with the oncolytic adenovirus, and wherein the oncolytic adenovirus is an oncolytic adenovirus with E1A polypeptide that cannot bind Rb, and comprises a fiber protein with an RGD amino acid inserted in the HI domain. Optionally, two or more of (i)-(iii) are observed or all three of (i)-(iii) are observed. The subject may also exhibit an autoimmune response against said glioma. The tumor response may include less defined tumor borders as determined by contrast MRI.

The oncolytic adenovirus may be a Δ24 adenovirus. A method of identification of a patient having a glioma may comprise tumor imaging, and said method may further comprise obtaining a biopsy of said tumor after identification of a patient having glioma and before treatment with the oncolytic adenovirus. The glioma may be an astrocytoma, an oligodendroglioma, an anaplastic glioma, a glioblastoma, an ependymoma, a meningioma, a pineal region tumor, a choroid plexus tumor, a neuroepithelial tumor, an embryonal tumor, a peripheral neuroblastic tumor, a tumor of cranial nerves, a tumor of the hemopoietic system, a germ cell tumor, or a tumor of the sellar region. The glioma may be recurrent, and/or the glioma may be failed one or more primary glioma therapies.

The glioma may be resectable, or not resectable. The glioma may be resected following said treatment. The post-resection tumor bed may be treated with said oncolytic adenovirus. The glioma may be contacted with the adenovirus by delivery of the adenovirus intracranially into the patient. The delivery may comprise intratumoral injection, may comprise multiple injections, such as where a post-resection catheter is implanted into said patient and said oncolytic adenovirus is delivered via said catheter. The oncolytic adenovirus may be administered via slow infusion over a period of minimum 10 minutes with a needle. The oncolytic adenovirus may be administered at stereotactly into more than one site in a glioma in said patient. The dose may be about 10³ to about 10¹⁵ viral particles, about 10⁵ to about 10¹² viral particles are administered to the patient, or about 10⁷ to about 10¹⁰ viral particles administered to the patient. The treatment may comprise dosing at 1 × 10⁷, 3 × 10⁷, 1 × 10⁸, 3 × 10⁸, 1 × 10⁹, 3 × 10⁹, 1 × 10¹⁰, and 3 × 10¹⁰ viral particles, including a dose escalation.

The method may further comprise administering to the patient a second therapy, wherein the second therapy is anti-angiogenic therapy, chemotherapy, immunotherapy, surgery, radiotherapy, immunosuppresive agents, or gene therapy with a therapeutic polynucleotide. The second therapy may be administered to the patient before administration of the composition comprising the oncolytic adenovirus, administered to the patient at the same time as administration of the composition comprising the oncolytic adenovirus, or administered to the patient after administration of the composition comprising the oncolytic adenovirus. The chemotherapy may comprise an alkylating agent, mitotic inhibitor, antibiotic, or antimetabolite. The second therapy may in particular comprise radiotherapy and temozolomide.

The subject may be further selected based on the presence of a Th1 response. The Th1 response may be is characterized by an increase in antigen-specific interferon-gamma (IFN-γ), IL-12, and complement-fixing antibodies.

In another embodiment, there is provided an oncolytic adenovirus for use in one or more of: (i) a method for treating a glioma in a human patient and achieving a clinical benefit in 30% of said patients, with clinical benefit defined by complete responders + partial responders plus stable disease; (ii) a method for treating a glioma in a human patient and achieving a 25% six-month progression-free survival; and (iii) a method for treating a glioma in a human patient and achieving a 12 month median survival for responders, with responders defined by complete responders + partial responders, wherein the method comprises contacting the gliomas of a population of the human patients that have been identified as having glioma with the oncolytic adenovirus, and wherein the oncolytic adenovirus is an oncolytic adenovirus with E1A polypeptide that cannot bind Rb, and comprises a fiber protein with an RGD amino acid inserted in the HI domain.

The oncolytic adenovirus may be a Δ24 adenovirus. Step (a) may comprise tumor imaging, and said method may further comprise obtaining a biopsy of said tumore after step (a) and before step (b). The glioma may be an astrocytoma, an oligodendroglioma, an anaplastic glioma, a glioblastoma, an ependymoma, a meningioma, a pineal region tumor, a choroid plexus tumor, a neuroepithelial tumor, an embryonal tumor, a peripheral neuroblastic tumor, a tumor of cranial nerves, a tumor of the hemopoietic system, a germ cell tumor, or a tumor of the sellar region. The glioma may be recurrent, and/or the glioma may be failed one or more primary glioma therapies.

The glioma may be resectable, or not resectable. The may be glioma resected following said treatment. The post-resection tumor bed may be treated with said oncolytic adenovirus. The glioma may be contacted with the adenovirus by delivery of the adenovirus intracranially into the patient. The delivery may comprise intratumoral injection, may comprise multiple injections, such as where a post-resection catheter is implanted into said patient and said oncolytic adenovirus is delivered via said catheter. The oncolytic adenovirus may be administered via slow infusion over a period of minimum 10 minutes with a needle. The oncolytic adenovirus may be administered at stereotactly into more than one site in a glioma in said patient. The dose may be about 10³ to about 10¹⁵ viral particles, about 10⁵ to about 10¹² viral particles are administered to the patient, or about 10⁷ to about 10¹⁰ viral particles administered to the patient.

The method may further comprise administering to the patient a second therapy, wherein the second therapy is anti-angiogenic therapy, chemotherapy, immunotherapy, surgery, radiotherapy, immunosuppresive agents, or gene therapy with a therapeutic polynucleotide. The second therapy may be administered to the patient before administration of the composition comprising the oncolytic adenovirus, administered to the patient at the same time as administration of the composition comprising the oncolytic adenovirus, or administered to the patient after administration of the composition comprising the oncolytic adenovirus. The chemotherapy may comprise an alkylating agent, mitotic inhibitor, antibiotic, or antimetabolite.

The subject may be further selected based on the presence of a Th1 response. The Th1 response may be is characterized by an increase in antigen-specific interferon-gamma (IFN-γ), IL-12, and complement-fixing antibodies.

Embodiments discussed in the context of a methods and/or composition of the invention may be employed with respect to any other method or composition described herein. Thus, an embodiment pertaining to one method may be applied to other methods of the invention as well.

The term "about" refers to the imprecision of determining virus, protein or other amounts and measures, and is intended to include at least one standard deviation of error for any particular assay, measure or quantification.

"A" or "an," as used herein in the specification, may mean one or more than one. As used herein in the claim(s), when used in conjunction with the word "comprising," the words "a" or "an" may mean one or more than one.

Other objects, features and advantages of the present invention will become apparent from the following detailed description. It should be understood, however, that the detailed description and the specific examples, while indicating preferred embodiments of the invention, are given by way of illustration only, since various changes and modifications within the scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein:

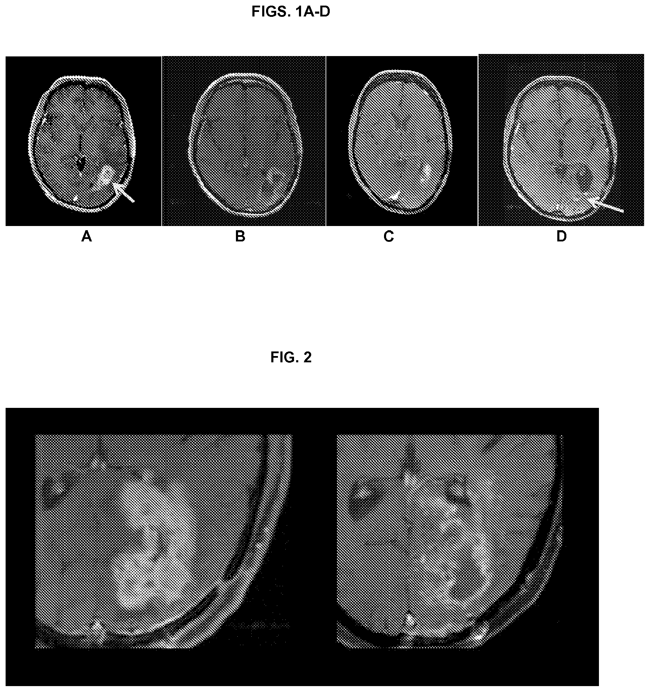

**FIGS. 1A-D**. Axial Contrast Images: Subject was treated with DNX-2401 after a first recurrence, having failed surgery, radiotherapy, Temozolomide and one cycle of Dasatinib. Currently no evidence of disease 32 months post treatment with a complete response to DNX-2401 therapy (by McDonald criteria). (FIG. 1A) Pretreatment, (FIG. 1B) 2 months, (FIG. 1C) 8 months, (FIG. 2D) 23 months. Arrow: Tumor. Note apparent progression in FIG. 1B caused by inflammation, not tumor growth. Tumor continues to respond (FIG. 1C), becoming smaller and appears fibrillar. Note absence of tumor in FIG. ID. Small enhancing region below the sulcus is a cyst (arrow).
**FIG. 2****. Axial Contrast Images:** Subject was treated with DNX-2401 at 3^{rd} recurrence having failed prior surgery, radiotherapy, Temozolomide and Bevacizumab. Note lobular appearance of tumor 2 months post Delta-24-RGD treatment (left panel) continuing on to evidence of disintegration ("soap bubbles") at 6 months (right panel). Tumor was resected at 6 months and analyzed. Independent pathology report stated that the tumor was mostly necrotic with the remainder infiltrated by immune cells with a predominance of T cells (left 2 months, right 6 months).

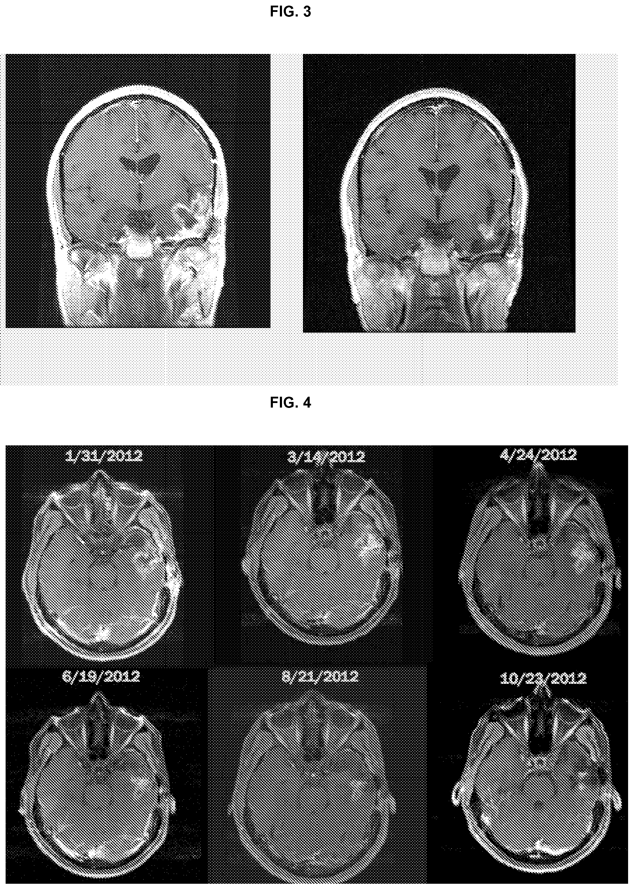

**FIG. 3****. Coronal Contrast Images (Right):** Subject was treated in the A arm of the trial with DNX-2401 at 1^{st} recurrence having failed prior surgery, radiotherapy, and temozolomide. Left image 1 month, right image 10 months post DNX-2401 treatment. Tumor volume reduced by 82% at 10 months
**FIG. 4****. Axial Contrast Images (Right):** Subject was treated in the A arm of the trial with DNX-2401 at 1^{st} recurrence having failed prior surgery, radiotherapy, and temozolomide.

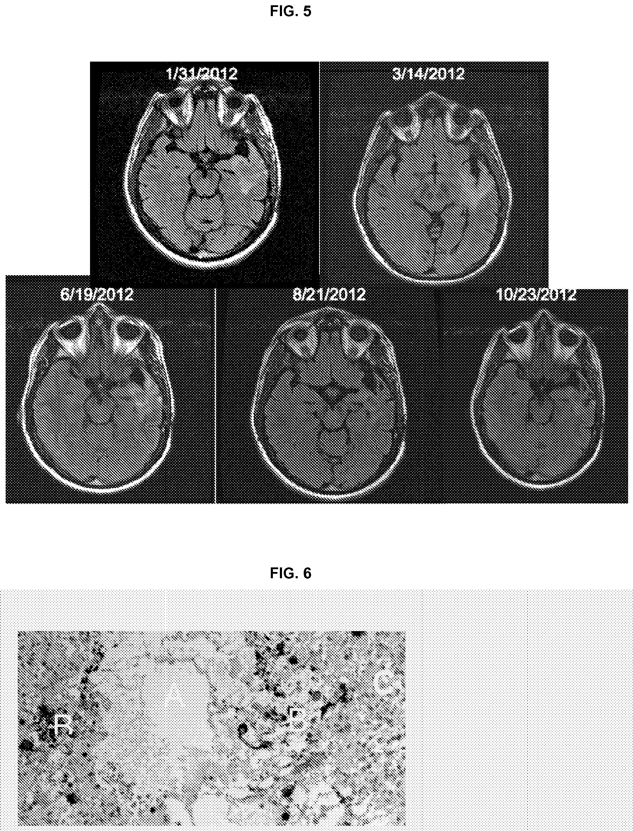

**FIG. 5****. T2/FLAIR Images:** Subject was treated in the A arm of the trial with DNX-2401 at 1^{st} recurrence having failed prior surgery, radiotherapy, and temozolomide. Images demonstrate profound improvement with virtually complete resolution of FLAIR signal.
**FIG. 6****. Sections through a human tumor resected from a Phase 1, B arm patient.** Stained for Ad hexon protein show clear evidence of virus spread and anti-glioma effects by 2 weeks post treatment with DNX-2401. A, Virus-induced necrosis; B, Infected tumor cells; C, uninfected tumor cells.

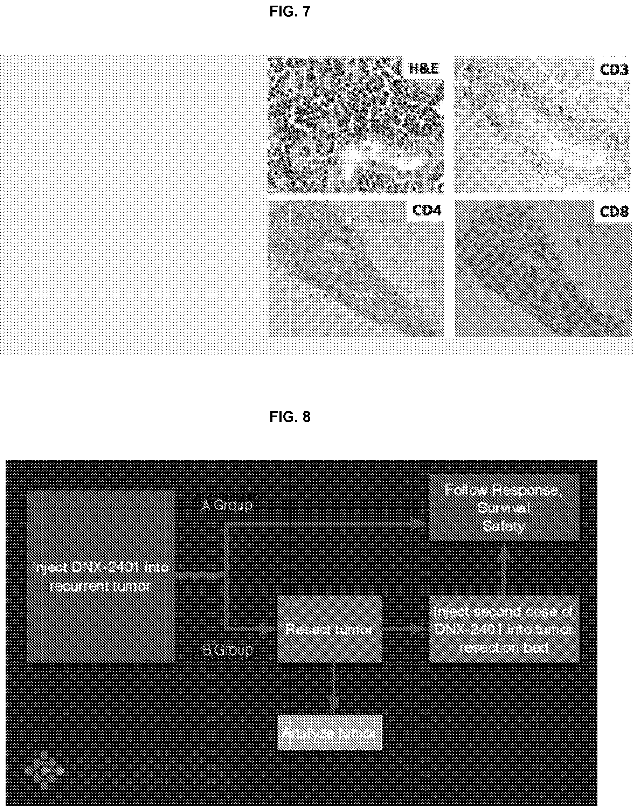

**FIG. 7****. Sections through a human tumor resected from a Phase 1, B arm patient.** Stained for the presence of T cells as shown. Note infiltration of predominantly CD8 T cells. H&E, hematoxylin/eosin; CD3, T cell specific marker normally present in resting and active T lymphocytes; CD4, T cell marker expressed in a helper/inducer T lymphocyte; CD8, T cell marker usually present on the cytotoxic/suppressor T cell subset.
**FIG. 8****. Phase I clinical trial design.**

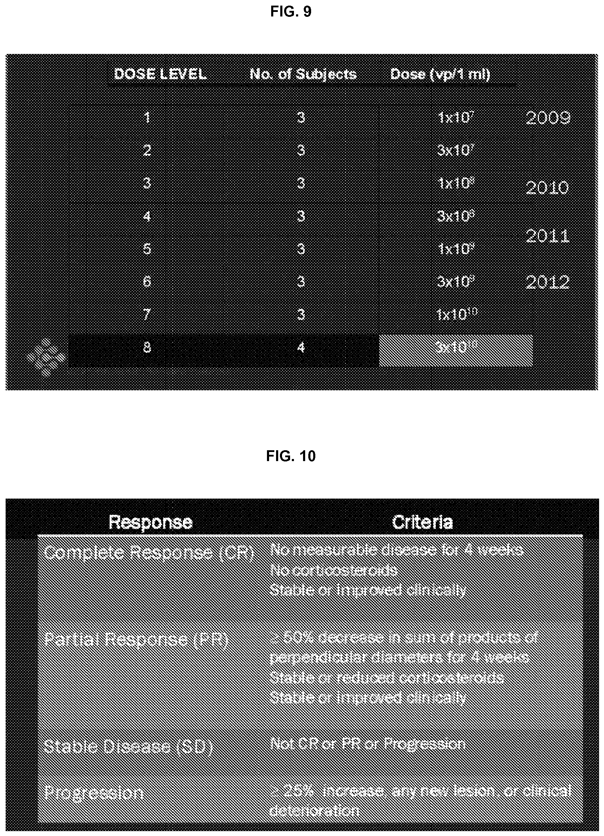

**FIG. 9****. Clinical dose study escalation plan.**
**FIG. 10****. Response criteria.**

### DESCRIPTION OF ILLUSTRATIVE EMBODIMENTS

Malignant tumors that are intrinsically resistant to conventional therapies are significant therapeutic challenges. Such malignant tumors include, but are not limited to malignant gliomas, which are the most abundant primary brain tumors having an annual incidence of 6.4 cases per 100,000 (CBTRUS, 2002-2003). These neurologically devastating tumors are the most common subtype of primary brain tumors and are one of the deadliest human cancers. In the most aggressive cancer, manifestation glioblastoma multiforme (GBM), median survival duration for patients ranges from 9 to 12 months, despite maximum treatment efforts (Hess *et al*., 1999). A prototypic disease, malignant glioma is inherently resistant to current treatment regimens (Shapiro and Shapiro, 1998). In fact, in approximately 1/3 of patients with GBM the tumor will continue to grow despite treatment with radiation and chemotherapy. Median survival even with aggressive treatment including surgery, radiation, and chemotherapy is less than 1 year (Schiffer, 1998). Because few good treatment options are available for many of these refractory tumors, the exploration of novel and innovative therapeutic approaches is essential.

One potential method to improve treatment is based on the concept that naturally occurring viruses can be engineered to produce an oncolytic effect in tumor cells (Wildner, 2001; Jacotat, 1967; Kirn, 2001; Geoerger *et al*., 2002; Yan *et al*., 2003; Vile *et al*., 2002). In the case of adenoviruses, specific deletions within their adenoviral genome can attenuate their ability to replicate within normal quiescent cells, while they retain the ability to replicate in tumor cells. One such conditionally replicating adenovirus, Δ24, has been described by Fueyo *et al.* (2000), see also U.S. Patent Publication No. 20030138405, and U.S. Patents 8,168,168 and 6,824,771). The Δ24 adenovirus is derived from adenovirus type 5 (Ad-5) and contains a 24-base-pair deletion within the CR2 portion of the E1A gene. Significant antitumor effects of Δ24 have been shown in cell culture systems and in malignant glioma xenograft models.

Oncolytic adenoviruses include conditionally replicating adenoviruses (CRADs), such as Delta 24, which have several properties that make them candidates for use as biotherapeutic agents. One such property is the ability to replicate in a permissive cell or tissue, which amplifies the original input dose of the oncolytic virus and helps the agent spread to adjacent tumor cells providing a direct antitumor effect.

### I. ONCOLYTIC ADENOVIRUS Δ24

The *in vitro* and *in vivo* oncolytic effects of Δ24 adenovirus have been demonstrated. Generally, adenovirus is a 36 kb, linear, double-stranded DNA virus (Grunhaus and Horwitz, 1992). Adenoviral infection of host cells results in adenoviral DNA being maintained episomally, which reduces the potential genotoxicity associated with integrating vectors. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in humans.

A particular form of the Δ24 virus is DNX-2401 (DNATrix, Houston TX) is a conditionally-replicating adenovirus (AdV) vector type 5 for intratumoral administration that contains a 24 bp deletion (bp 923-946; the Rb-binding domain) in the E1A gene and the insertion of an RGD integrin-binding motif (4C peptide: Cys-Asp-Cys-Arg-Gly-Asp-Cys-Phe-Cys; SEQ ID NO: 1) in the HI loop of the Ad fiber. DNX-2401 can use certain cell surface integrins to gain entry to tumor cells.

DNX-2401 enters cells via both the normal adenovirus receptor (CAR) as well as the RGD-binding integrins normally expressed only on tumor cells and neovasculature. This is a significant improvement upon previous generation adenoviruses that had to rely on the CAR receptor for activity. This RGD-4C peptide (CDCRGDCFC; SEQ ID NO: 1) has been shown to bind with high affinity to the RGD-binding (αVβ3 and αVβ5) integrins present on the surface of many cell types, including tumor cells. Importantly, RGD-binding integrins have been shown to be expressed in tumor vasculature and on glioma cells but not in normal brain, thereby providing a basis for greatly increased and selective infection of glioblastoma by DNX-2401.

Once inside the cell, DNX-2401 replication is restricted to cells with defects in the Rb pathway, the primary control pathway for cellular division. Because virtually all tumor cells, including >90% of glioblastomas, are defective in the Rb/p16 pathway and already in the cell cycle, DNX-2401 replicates in and kills these tumor cells selectively and efficiently. This high degree of selectivity is accomplished by the deletion of the 24 bp Rb binding domain normally present in the virus E1 protein. A major function of this region is to enable adenovirus replication in healthy cells that have normal Rb function. The deletion of this region causes DNX-2401 to be able to replicate only in tumor cells with Rb pathway defects.

### A. Mechanism of Δ24 Oncolytic Virus

A dramatic increase in the cellular proliferation that is characteristic of the transformation from low-grade to intermediate-grade glioma is in large part related to dysregulation of the p16/Rb/E2F pathway (Fueyo *et al*., 2000; Fueyo *et al*., 1998; Chintala, 1997). Most compelling is the lack of mutational overlap seen among the various members of this pathway, which argues that an important therapeutic advance in the treatment of these tumors could be achieved by specifically targeting the Rb pathway (Kyritsis and Yung, 1996; Fueyo *et al*., 1999). Disrupted Rb status will likely provide opportunities to utilize agents that operate exclusively in Rb -deficient tumor cells (Fueyo *et al*., 1999). Most normal human brain cells are usually quiescent. Cells in the central nervous system (CNS) rarely divide, and these cells are specifically triggered to divide in a limited fashion. Tight regulatory controls have evolved which strictly limit cells from undergoing cell division. The p16/Rb/E2F pathway is an important pathway for maintaining the non-dividing status of fully differentiated cell or negatively regulates the cell-cycle progression of dividing normal cells.

Human adenovirus normally infects human cells, which are quiescent (nondividing) or dividing cells (normal or cancer cells). Upon introduction of this virus into a human cell (viral infection), the adenovirus DNA is immediately transcribed by the synthesis of E1A adenoviral protein. The CR2 region of E1A protein interacts specifically with Rb protein and leads to release of E2F, forcing cell entry into S-phase (the DNA Synthesis phase) of the cell cycle and maintaining the cell in the dividing cycle. This series of events effectively commandeers the host cell exclusively for the purpose of expressing virally encoded proteins. Active production of adenoviral particles depends on this ability to drive cells into an active mode of replication, a critical feature of oncolytic viruses. As a consequence of their biologic characteristics, tumor cells provide a replicating environment that favors such activity. Mutations in critical sequences of the viral genome render the adenovirus unable to bind to and inactivate tumor suppressor proteins. These modified adenoviruses are able to replicate exclusively in cells lacking a functional target tumor suppressor gene (tumor cells only).

Thus, the expression of an E1A protein with a 24 base pair deletion in the CR2 region prevents the protein from binding to and inactivating Rb. This attenuated E1A-mutant adenovirus is unable to replicate within normal quiescent cells that have a funtionally active Rb pathway. In contrast, tumor cells are permissive to viral replication, which in turn efficiently invade and lyse human glioma cells both *in vitro* and *in vivo.*

The oncolytic potential of Δ24 is dramatic compared with other conditionally replication-deficient adenoviruses, such as Onyx-015. The effects of Δ24 in a mouse xenograft intracranial glioma tumor model are shown in FIG 2. In this case, the curve representing RA55 carries the deletion in the E1B region as in Onyx-015. The oncolytic adenovirus does not have the same degree of potency as Δ24 at comparable doses used (in this case 1 x 10⁸ pfu). Also shown is the negative control Δ24 that is inactivated by ultraviolet exposure. The antitumor effects of Δ24 have been demonstrated in various human tumor cell lines and in animal xenograft models with known defects of the p16/Rb/E2F pathway. Permissive replication of Δ24 in cell lines with p16/Rb/E2F defects is contrasted with the highly attenuated replication in normal astrocytes and normal quiescent fibroblasts. Additionally, the activity of this virus is attenuated when introduced into tumor cells in which Rb has been functionally restored through stable or transient transfection techniques.

Several factors favor the use of oncolytic adenoviruses for the treatment of brain tumors. First, gliomas do not metastasize, and therefore an efficient local approach should be enough to cure the disease. Second, every glioma harbors several populations of cells expressing different genetic abnormalities (Sidransky *et al*., 1992; Collins and James, 1993; Furnari *et al*., 1995; Kyritsis *et al*., 1996). Thus, the spectrum of tumors sensitive to the transfer of a single gene to cancer cells may be limited. Third, replication competent adenoviruses can infect and destroy cancer cells that are arrested in G₀. Since gliomas invariably include non-cycling cells, this property is important. Finally, the pl6-Rb pathway is abnormal in the majority of gliomas (Hamel *et al*., 1993; Henson *et al*., 1994; Hirvonen *et al*., 1994; Jen *et al*., 1994; Schmidt *et al*., 1994; Costello *et al*., 1996; Fueyo *et al*., 1996b; Kyritsis *et al*., 1996; Ueki *et al*., 1996; Costello *et al*., 1997), thus making the Δ24 strategy appropriate for most of these tumors. Although the loss of the retinoblastoma tumor suppressor gene function has been associated with the causes of various types of tumors and is not limited to treatment of gliomas.

In other embodiments of the invention, an E1A mutation (e.g., a Δ24 mutation in E1A) may be used in combination with mutations in the E1B region of the same adenovirus, thus producing a double mutant adenovirus. In certain embodiments of the invention an adenovirus may comprise a Δ24 mutation and a deletion in the E1B region that prevents expression or function of the E1B55kD protein. The E1B55kD protein has been shown to bind to and inactivate p53. The E1B region mutation may include a deletion of adenovirus sequences from 2426bp to 3328bp of genebank accession number NC_001406, which is incorporated herein by reference.

In certain embodiments of the invention, an oncolytic adenovirus may be used as an adenovirus expression vector. "Adenovirus expression vector" is meant to include those vectors containing adenovirus sequences sufficient to (a) support packaging of the vector and (b) to express a polynucleotide that has been cloned therein. The insertion position of a polynucleotide encoding a heterologous polypeptide of interest within the adenovirus sequences is not critical to the invention. The polynucleotide encoding the polypeptide of interest may be inserted in lieu of the deleted E3 region in E3 replacement vectors as described by Karlsson *et al*., (1986) or other region that are not essential for viral replication in the target cell. Traditional methods for the generation of adenoviral particles is co-transfection followed by subsequent *in vivo* recombination of a shuttle plasmid and an adenoviral helper plasmid into either 293 or 911 cells (Introgene, The Netherlands).

### B. Neoplastic Cell Surface Integrin Targeting

Modifications of oncolytic adenovirus described herein may be made to improve the ability of the oncolytic adenovirus to treat cancer. The present invention also includes any modification of oncolytic adenovirus that improves the ability of the adenovirus to target neoplastic cells. Included are modifications to oncolytic adenovirus genome in order to enhance the ability of the adenovirus to infect and replicate in cancer cells by altering the receptor binding molecules.

Cell surface receptors are attractive candidates for the targeted therapy of cancer. The absence or the presence of low levels of the coxsackievirus and adenovirus receptor (CAR) on several tumor types can limit the efficacy of the oncolytic adenovirus. Various peptide motifs may be added to the fiber knob, for instance an RGD motif (RGD sequences mimic the normal ligands of cell surface integrins), Tat motif, poly-lysine motif, NGR motif, CTT motif, CNGRL motif, CPRECES motif or a strept-tag motif (Rouslahti and Rajotte, 2000). A motif can be inserted into the HI loop of the adenovirus fiber protein. Modifying the capsid allows CAR-independent target cell infection. This allows higher replication, more efficient infection, and increased lysis of tumor cells (Suzuki *et al*., 2001). Peptide sequences that bind specific human glioma receptors such as EGFR or uPR may also be added. Specific receptors found exclusively or preferentially on the surface of cancer cells may used as a target for adenoviral binding and infection, such as EGFRvIII.

### II. RB PATHWAY

Rb is a tumor suppressor gene whose loss of function is associated with tumor formation. Retinoblastoma protein or Rb, as used herein, refers to the polypeptide encoded by the retinoblastoma gene (Rb). The retinoblastoma gene is located at 13ql4 in humans and encodes a protein of approximately 110 kiloDaltons (kD). Unphosphorylated Rb inhibits cell proliferation by sequestering transcription factors (e.g., E2F) and arresting cells in G₁ of the cell cycle. Transcription factors are released from Rb when Rb is phosphorylated. The binding of E1A to Rb causes transcriptional factor release in much the same manner as phosphorylation. Several viral oncoproteins target Rb for inactivation in order to facilitate viral replication. These proteins include adenovirus E1A, SV40 large T antigen, and papillomavirus E7.

The E1A protein is one of the first virus-specific polypeptides synthesized after adenoviral infection and is required for viral replication to occur (Dyson and Harlow, 1992; Flint and Shenk, 1997). Interaction of the Rb protein and the E1A protein results in release of E2F from pre-existing cellular E2F-Rb complexes. E2F is then free to activate transcription from E2 promoters of adenovirus and E2F regulated genes of an infected cell. The transcriptional activation of these cellular genes in turn helps to create an environment suitable for viral DNA synthesis in otherwise quiescent cells (Nevins, 1992). Two segments of E1A are important for binding Rb; one includes amino acids 30-60 and the other amino acids 120-127 (Whyte *et al*., 1988; Whyte *et al*., 1989). Deletion of either region prevents the formation of detectable ElA/Rb complexes *in vitro* and *in vivo* (Whyte *et al*., 1989).

An adenovirus containing a Delta 24 mutation produces an E1A protein that cannot bind Rb, causing an infected cell to remain in G₀. Thus a mutant Rb pathway and a mutant E1A, along with E2F activation are necessary for Δ24 adenoviral transcription.

Retinoblastoma (Rb) pathway, as used herein, refers the interaction of a group of regulatory proteins that interact with Rb or other proteins that interact with Rb in regulating cell proliferation (for review see Kaelin, 1999). Proteins within the Rb pathway include, but are not limited to, Rb, the E2F family of transcription factors, DRTF, RIZ286, MyoD287, cAb1288, MDM2289, hBRG1/hBRM, pl6, p107, pl30, c-Abl tyrosine kinase and proteins with conserved LXCXE motifs, cyclin E-cdk 2, and cyclin D-cdk 4/6. Phosphorylation of Rb releases E2F, which is bound to unphosphorylated Rb. E2F stimulates cyclin E transcription and activity, which results in more Rb phosphorylation. Unphosphorylated Rb acts as a tumor suppressor by binding to regulatory proteins that increase DNA replicaiton, such as E2F (The Genetic Basis of Human Cancer, Vogelstein and Kinzler eds., 1998).

Defective retinoblastoma pathway, as used herein, refers to inactivation, mutation, or deletion of the Rb or the inability of the upstream or downstream regulatory proteins that interact with Rb to regulate cell proliferation due to a mutation or modification of one or more proteins, protein activities, or protein-protein interactions. Mutations causing a defective Rb pathway include, but are not limited to inactivating mutations in Rb, INK4 proteins, and CIP/KIP and activating mutations in the cyclin genes, such as cyclin D/cdk 4, 6 and cyclin E, cdk 2. Mutations in one or another element of the Rb regulatory pathway, including p16, cyclin D, cdk4, E2F or Rb itself, may be mutated in almost 100 percent of human tumors (The Genetic Basis of Human Cancer, 1998). Rb associated tumors include gliomas, sarcomas, tumors of the lung, breast, ovary, cervix, pancreas, stomach, colon, skin, larynx, bladder and prostate.

### III. METHODS FOR TREATING BRAIN CANCERS

The present invention involves the treatment of brain tumors, including tumor cells with a disrupted Rb pathway. It is contemplated that a wide variety of brain tumors may be treated using the methods and compositions of the invention, including glioblastoma, anaplastic astrocytoma, and gliosarcoma.

The term "glioma" refers to a tumor originating in the neuroglia of the brain or spinal cord. Gliomas are derived form the glial cell types such as astrocytes and oligodendrocytes, thus gliomas include astrocytomas and oligodendrogliomas, as well as anaplastic gliomas, glioblastomas, and ependymomas. Astrocytomas and ependymomas can occur in all areas of the brain and spinal cord in both children and adults. Oligodendrogliomas typically occur in the cerebral hemispheres of adults. Gliomas account for 75% of brain tumors in pediatrics and 45% of brain tumors in adults. The remaining percentages of brain tumors are meningiomas, ependymomas, pineal region tumors, choroid plexus tumors, neuroepithelial tumors, embryonal tumors, peripheral neuroblastic tumors, tumors of cranial nerves, tumors of the hemopoietic system, germ cell tumors, and tumors of the sellar region.

For the purpose of this document, response and progression criteria (all responses durable for at least 4 weeks) are defined as those terms were adopted by the World Health Organization and adapted for brain tumors, using Macdonald criteria (Macdonald *et al*., 1990), and are determined using bi-dimensional measurements of contrast-enhancing lesions (reduction on longest cross diameter of a lesion on an MRI scan):
- Complete response: disappearance of all lesions and no steroids above physiologic dose;
- Partial response: ≥ 50% shrinkage and stable or decreased steroids;
- Stable disease: <50% shrinkage;
- Progression: new lesion, unequivocal progression of nonindex lesions, ≥ 25% growth of index lesions, or clear clinical deterioration in the absence of radiologic progression.

Glioblastoma is a devastating primary high grade malignant glioma resistant to conventional therapies. Current intervention, such as surgery, radiotherapy and chemotherapy, extends overall median survival to approximately 14.6 months. Many new compounds, even when tested in combination, have failed to improve overall survival or lead to a useful clinical response. There is a profound unmet medical need for a new mode of attack on these tumors that can impact the course of disease.

High-grade malignant gliomas are highly vascular and infiltrative tumors, and are therefore inclined to recur despite surgical resection. Treatment options are limited for newly-diagnosed as well as recurrent disease, and are especially limited for patients who have tumors that are not surgically accessible. Furthermore, while 80% or more of glioblastoma recurrences occur in the same area as the original tumor, additional radiation therapy is often precluded because of toxicity concerns. Temozolomide is approved for treating newly-diagnosed glioblastoma and recurrent anaplastic astrocytoma and bevaciumab was more recently approved for treating recurrent glioblastoma. These drugs are systemically delivered, and must be administered as a multi-dose regimen. Temozolomide is used most effectively as an adjuvant to surgery or radiotherapy. By contrast, bevacizumab is often administered on its own for recurrent disease, or experimentally, in combination with existing chemotherapies such as irinotecan.

Progress in understanding tumor biology has allowed the identification of a number of key signaling pathways and processes of tumorigenesis. However, owing to the redundancy of pathways and alternative signaling, inhibition of a single target may be insufficient to substantially inhibit tumor growth, and a combination of several agents may be needed.

Currently, there are numerous anti-angiogenic agents being considered in clinical practice. Because of its accelerated FDA approval, the anti-angiogenic drug most commonly investigated in patients with brain tumors is bevacizumab (Avastin®), which is a humanized monoclonal antibody that disrupts the VEGF pathway, induces a decrease in tumor vessel size, and results in a more normalized vascular network that has reduced permeability. This compound has now been used in a number of studies as both a single and combined agent, in upfront and recurrent settings.

The majority of current ongoing phase II/III studies have transitioned to using small molecule kinase or integrin inhibitors, such as enzastaurin, cediranib, pazopanib, sorafenib, sunitinib, and cilengitide. The therapeutic regimens may include a combination of these therapies, often involve the concurrent prescription of non - anti-angiogenic treatments, and can be administered to both patients with newly diagnosed and recurrent disease. Initial results suggests that while several of these agents can modestly prolong 6-month PFS, the potential long-term benefits and impact on survival remain to be demonstrated.

Glioblastoma multiforme is the most common malignant primary brain tumor of adults. More than half of these tumors have abnormalities in genes involved in cell cycle control. Often there is a deletion in the CDKN2A or a loss of expression of the retinoblastoma gene. Other types of brain tumors include astrocytomas, oligodendrogliomas, ependymomas, medulloblastomas, meningiomas and schwannomas.

In many contexts, it is not necessary that the cell be killed or induced to undergo cell death or "apoptosis." Rather, to accomplish a meaningful treatment, all that is required is that the tumor growth be slowed to some degree. It may be that the cell's growth is completely blocked or that some tumor regression is achieved. Clinical terms such as "remission" and "reduction of tumor" burden also are contemplated given their normal usage.

The term "therapeutic benefit" refers to anything that promotes or enhances the well-being of the subject with respect to the medical treatment of his/her condition, which includes treatment of pre-cancer, cancer, and hyperproliferative diseases. A list of nonexhaustive examples of this includes extension of the subject's life by any period of time, decrease or delay in the neoplastic development of the disease, decrease in hyperproliferation, reduction in tumor growth, delay of metastases, reduction in cancer cell or tumor cell proliferation rate, and a decrease in pain to the subject that can be attributed to the subject's condition.

### A. Adenoviral Therapies

Those of skill in the art are well aware of how to apply adenoviral delivery to *in vivo* and *ex vivo* situations. For viral vectors, one generally will prepare a viral vector stock. Depending on the kind of virus and the titer attainable, one will deliver 1 to 100, 10 to 50, 100-1000, or up to 1 x 10⁴, 1 x 10⁵, 1 x 10⁶, 1 x 10⁷, 1 x 10⁸, 1 x 10⁹, 1 x 10¹⁰, 1 x 10¹¹, 1 x 10¹² or 1 x 10¹³ infectious particles to the patient in a pharmaceutically acceptable composition as discussed below.

Various routes are contemplated for various tumor types. Where discrete tumor mass, or solid tumor, may be identified, a variety of direct, local and regional approaches may be taken. For example, the tumor may be directly injected with the adenovirus. A tumor bed may be treated prior to, during or after resection and/or other treatment(s). Following resection or other treatment(s), one generally will deliver the adenovirus by a catheter having access to the tumor or the residual tumor site following surgery. One may utilize the tumor vasculature to introduce the vector into the tumor by injecting a supporting vein or artery. A more distal blood supply route also may be utilized.

The method of treating cancer includes treatment of a tumor as well as treatment of the region near or around the tumor. In this application, the term "residual tumor site" indicates an area that is adjacent to a tumor. This area may include body cavities in which the tumor lies, as well as cells and tissue that are next to the tumor.

### B. Formulations and Routes of Administration to Patients

Where clinical applications are contemplated, it will be necessary to prepare pharmaceutical compositions in a form appropriate for the intended application. Generally, this will entail preparing compositions that are essentially free of pyrogens, as well as other impurities that could be harmful to humans or animals.

The active compositions of the present invention may include classic pharmaceutical preparations. One will generally desire to employ appropriate salts and buffers to render delivery vectors stable and allow for uptake by target cells. Aqueous compositions of the present invention comprise an effective amount of the vector to cells, dissolved or dispersed in a pharmaceutically acceptable carrier or aqueous medium. Such compositions also are referred to as inocula. The phrase "pharmaceutically or pharmacologically acceptable" refers to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the present invention, its use in therapeutic compositions is contemplated. Supplementary active ingredients also can be incorporated into the compositions.

Administration of these compositions according to the present invention will be via an appropriate route, but are particularly drawn to intracranial/intratumoral administration. Administration may be by injection or infusion, see Kruse *et al.* (1994), specifically incorporated by reference, for methods of performing intracranial administration. Such compositions would normally be administered as pharmaceutically acceptable compositions.

An effective amount of the therapeutic agent is determined based on the intended goal, for example, elimination of tumor cells. The term "unit dose" refers to physically discrete units suitable for use in a subject, each unit containing a predetermined-quantity of the therapeutic composition calculated to produce the desired responses, discussed above, in association with its administration, *i.e.,* the appropriate route and treatment regimen. The quantity to be administered, both according to number of treatments and unit dose, depends on the subject to be treated, the state of the subject and the protection desired. Precise amounts of the therapeutic composition also depend on the judgment of the practitioner and are peculiar to each individual. The engineered viruses of the present invention may be administered directly into animals, or alternatively, administered to cells that are subsequently administered to animals.

As used herein, the term *in vitro* administration refers to manipulations performed on cells removed from an animal, including, but not limited to, cells in culture. The term *ex vivo* administration refers to cells that have been manipulated *in vitro,* and are subsequently administered to a living animal. The term *in vivo* administration includes all manipulations performed on cells within an animal. In certain aspects of the present invention, the compositions may be administered either *in vitro, ex vivo,* or *in vivo.* An example of *in vivo* administration includes direct injection of tumors with the instant compositions by intracranial administration to selectively kill tumor cells.

Intratumoral injection or injection into the tumor vasculature is specifically contemplated for discrete, solid, accessible tumors including tumor exposed during surgery. For tumors 1.5 to 5 cm in diameter, the injection volume will be 1 to 3 cc, preferably 3 cc. For tumors greater than 5 cm in diameter, the injection volume will be 4 to 10 cc, preferably 5 cc. Multiple injections delivered as single dose comprise about 0.1 to about 0.5 ml volumes, preferable 0.2 ml. The viral particles may advantageously be contacted by administering multiple injections to the tumor, spaced at approximately 1 cm intervals.

In the case of surgical intervention, the present invention may be used preoperatively, to render an inoperable tumor subject to resection. Alternatively, the present invention may be used at the time of surgery, and/or thereafter, to treat residual or metastatic disease. For example, a resected tumor bed may be injected or perfused with a formulation comprising the adenovirus. The perfusion may be continued post-resection, for example, by leaving a catheter implanted at the site of the surgery. Periodic post-surgical treatment also is envisioned.

Continuous administration, preferably via catheterization, also may be applied where appropriate, for example, where a tumor is excised and the tumor bed is treated to eliminate residual, microscopic disease. Such continuous perfusion may take place for a period from about 1-2 hr, to about 2-6 hr, to about 6-12 hr, to about 12-24 hr, to about 1-2 days, to about 1-2 wk or longer following the initiation of treatment. Generally, the dose of the therapeutic composition via continuous perfusion will be equivalent to that given by a single or multiple injections, adjusted over a period of time during which the perfusion occurs.

Treatment regimens may vary as well, and often depend on tumor type, tumor location, disease progression, and health and age of the patient. Obviously, certain types of tumor will require more aggressive treatment, while at the same time, certain patients cannot tolerate more taxing protocols. The clinician will be best suited to make such decisions based on the known efficacy and toxicity (if any) of the therapeutic formulations.
Solutions of the active compounds as free base or pharmacologically acceptable salts can be prepared in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations contain a preservative to prevent the growth of microorganisms.

The therapeutic compositions of the present invention are advantageously administered in the form of injectable compositions either as liquid solutions or suspensions; solid forms suitable for solution in, or suspension in, liquid prior to injection may also be prepared. These preparations also may be emulsified. A typical composition for such purpose comprises a pharmaceutically acceptable carrier. For instance, the composition may contain 10 mg, 25 mg, 50 mg or up to about 100 mg of human serum albumin per milliliter of phosphate buffered saline. Other pharmaceutically acceptable carriers include aqueous solutions, non-toxic excipients, including salts, preservatives, buffers and the like. Examples of non-aqueous solvents are propylene glycol, polyethylene glycol, vegetable oil and injectable organic esters such as ethyloleate. Aqueous carriers include water, alcoholic/aqueous solutions, saline solutions, parenteral vehicles such as sodium chloride or Ringer's dextrose. Intravenous vehicles include fluid and nutrient replenishers. Preservatives include antimicrobial agents, anti-oxidants, chelating agents and inert gases. The pH and exact concentration of the various components the pharmaceutical composition are adjusted according to well known parameters. When the route is topical, the form may be a cream, ointment, or salve.

### C. Combination Therapy

Tumor cell resistance to various therapies represents a major problem in clinical oncology. One goal of current cancer research is to find ways to improve the efficacy of chemo- and radiotherapy, as well as other conventional cancer therapies. One way is by combining such traditional therapies with oncolytic adenovirus therapy. Traditional therapy to treat cancers may include removal of all or part of the affected organ, external beam irradiation, xenon arc and argon laser photocoagulation, cryotherapy, immunotherapy and chemotherapy. The choice of treatment is dependent on multiple factors, such as, 1) multifocal or unifocal disease, 2) site and size of the tumor, 3) metastasis of the disease, 4) age of the patient or 5) histopathologic findings (The Genetic Basis of Human Cancer, 1998).

In the context of the present invention, it is contemplated that adenoviral therapy could be used in conjunction with anti-cancer agents, including chemo- or radiotherapeutic intervention, as well as radiodiagnositc techniques. It also may prove effective to combine oncolytic virus therapy with immunotherapy.

A "target" cell contacting a mutant oncolytic virus and optionally at least one other agent may kill cells, inhibit cell growth, inhibit metastasis, inhibit angiogenesis or otherwise reverse or reduce a hyperproliferative phenotype of target cells. These compositions would be provided in a combined amount effective to kill or inhibit proliferation of the target cell. This process may involve contacting the cells with the expression construct and the agent(s) or factor(s) at the same or different times. This may be achieved by contacting the cell with a single composition or pharmacological formulation that includes both agents, or by contacting the cell with two distinct compositions or formulations, wherein one composition includes the oncolytic adenvirus and the other includes the second agent.

Oncolytic adenoviral therapy may also be combined with immunosuppression. The immunosuppression may be performed as described in WO 96/12406). Examples of immunosuppressive agents include cyclosporine, FK506, cyclophosphamide, and methotrexate.

Alternatively, an oncolytic adenovirus treatment may precede or follow the second agent or treatment by intervals ranging from minutes to weeks. In embodiments where the second agent and oncolytic adenovirus are applied separately to the cell, one would generally ensure that a significant period of time did not expire between the time of each delivery, such that the second agent and oncolytic adenovirus would still be able to exert an advantageously combined effect on the cell. In such instances, it is contemplated that one would contact the cell with both modalities within about 12-24 hr of each other and, more preferably, within about 6-12 hr of each other, with a delay time of only about 12 hours being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

It also is conceivable that more than one administration of either oncolytic adenovirus and/or the second agent will be desired. Various combinations may be employed, where oncolytic adenovirus is "A" and the other agent is "B", as exemplified below:
A/B/A B/A/B B/B/A A/A/B B/A/A A/B/B B/B/B/A B/B/A/B
A/A/B/B A/B/A/B A/B/B/A B/B/A/A B/A/B/A B/A/A/B B/B/B/A
A/A/A/B B/A/A/A A/B/A/A A/A/B/A A/B/B/B B/A/B/B B/B/A/B

Other combinations are contemplated. Again, to achieve cell killing, both agents are delivered to a cell in a combined amount effective to kill the cell.

Agents or factors suitable for use in a combined therapy are any anti-angiogenic agent and/or any chemical compound or treatment method with anticancer activity; therefore, the term "anticancer agent" that is used throughout this application refers to an agent with anticancer activity. These compounds or methods include alkylating agents, topoisomerase I inhibitors, topoisomerase II inhibitors, RNA/DNA antimetabolites, DNA antimetabolites, antimitotic agents, as well as DNA damaging agents, which induce DNA damage when applied to a cell.

Examples of chemotherapy drugs and pro-drugs include, CPT11, temozolomide, platin compounds and pro-drugs such as 5-FC. Examples of alkylating agents include, inter alia, chloroambucil, cis-platinum, cyclodisone, flurodopan, methyl CCNU, piperazinedione, teroxirone. Topoisomerase I inhibitors encompass compounds such as camptothecin and camptothecin derivatives, as well as morpholinodoxorubicin. Doxorubicin, pyrazoloacridine, mitoxantrone, and rubidazone are illustrations of topoisomerase II inhibitors. RNA/DNA antimetabolites include L-alanosine, 5-fluoraouracil, aminopterin derivatives, methotrexate, and pyrazofurin; while the DNA antimetabolite group encompasses, for example, ara-C, guanozole, hydroxyurea, thiopurine. Typical antimitotic agents are colchicine, rhizoxin, taxol, and vinblastine sulfate. Other agents and factors include radiation and waves that induce DNA damage such as, γ-irradiation, X-rays, UV-irradiation, microwaves, electronic emissions, and the like. A variety of anti-cancer agents, also described as "chemotherapeutic agents," function to induce DNA damage, all of which are intended to be of use in the combined treatment methods disclosed herein. Chemotherapeutic agents contemplated to be of use, include, e.g., adriamycin, bleomycin, 5-fluorouracil (5-FU), etoposide (VP-16), camptothecin, actinomycin-D, mitomycin C, cisplatin (CDDP), podophyllotoxin, verapamil, and even hydrogen peroxide. The invention also encompasses the use of a combination of one or more DNA damaging agents, whether radiation-based or actual compounds, such as the use of X-rays with cisplatin or the use of cisplatin with etoposide.

In treating pre-cancer or cancer according to the invention, one would contact the cells of a precancerous lesion or tumor cells with an agent in addition to the oncolytic adenovirus. This may be achieved by irradiating the localized tumor site with radiation such as X-rays, UV-light, γ-rays or even microwaves. Alternatively, the cells may be contacted with the agent by administering to the subject a therapeutically effective amount of a pharmaceutical composition comprising a compound such as adriamycin, bleomycin, 5-fluorouracil, etoposide, camptothecin, actinomycin-D, mitomycin C, podophyllotoxin, verapamil, or more preferably, cisplatin. The agent may be prepared and used as a combined therapeutic composition, or kit, by combining it with an oncolytic adenovirus.

Agents that directly cross-link nucleic acids, specifically DNA, are envisaged to facilitate DNA damage leading to a synergistic, anti-neoplastic combination with an oncolytic adenovirus. Cisplatinum agents such as cisplatin, and other DNA alkylating agents may be used. Cisplatin has been widely used to treat cancer, with efficacious doses used in clinical applications of 20 mg/m² for 5 days every three weeks for a total of three courses. Cisplatin is not absorbed orally and must therefore be delivered via injection intravenously, subcutaneously, intratumorally or intraperitoneally. Bleomycin and mitomycin C are other anticancer agents that are administered by injection intravenously, subcutaneously, intratumorally or intraperitoneally. A typical dose of bleomycin is 10 mg/m², while such a dose for mitomycin C is 20 mg/m².

Agents that damage DNA also include compounds that interfere with DNA replication, mitosis and chromosomal segregation. Such chemotherapeutic compounds include adriamycin, also known as doxorubicin, etoposide, verapamil, podophyllotoxin, and the like. Widely used in a clinical setting for the treatment of neoplasms, these compounds are administered through bolus injections intravenously at doses ranging from 25-75 mg/m² at 21 day intervals for adriamycin, to 35-50 mg/m² for etoposide intravenously or double the intravenous dose orally.

Agents that disrupt the synthesis and fidelity of nucleic acid precursors and subunits also lead to DNA damage. As such a number of nucleic acid precursors have been developed. Particularly useful are agents that have undergone extensive testing and are readily available. As such, agents such as 5-fluorouracil (5-FU), are preferentially used by neoplastic tissue, making this agent particularly useful for targeting to neoplastic cells. Although quite toxic, 5-FU, is applicable in a wide range of carriers, including topical, however intravenous administration with doses ranging from 3 to 15 mg/kg/day being commonly used or as alternative 5-FC may be administered and converted in a target tissue or target cell.

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage DNA, on the precursors of DNA, the replication and repair of DNA, and the assembly and maintenance of chromosomes. Dosage ranges for X-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells.

Immunotherapy may be used as part of a combined therapy, in conjunction with mutant oncolytic virus-mediated therapy. The general approach for combined therapy is discussed below. Generally, the tumor cell must bear some marker that is amenable to targeting, *i.e.,* is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155. Antibodies specific for CAR, integrin or other cell surface molecules, may be used to identify cells that the adenovirus could infect well. CAR is an adenovirus receptor protein. The penton base of adenovirus mediates viral attachment to integrin receptors and particle internalization.

The skilled artisan is directed to "Remington's Pharmaceutical Sciences" 15th Edition, 1980. Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

In addition to combining oncolytic adenovirus therapies with chemo- and radiotherapies, it also is contemplated that combination with other gene therapies will be advantageous. For example, targeting of an oncolytic adenovirus in combination with the targeting of p53 at the same time may produce an improved anti-cancer treatment. Any tumor-related gene or nucleic acid encoding a polypeptide conceivably can be targeted in this manner, for example, p21, Rb, APC, DCC, NF-1, NF-2, BCRA2, p16, FHIT, WT-1, MEN-I, MEN-II, BRCA1, VHL, FCC, MCC, ras, myc, neu, raf, erb, src, fms, jun, trk, ret, gsp, hst, bcl and abl.

Anti-angiogenic therapies may also be combined advantageously with the oncolytic adenovirus therapies disclosed herein. In particular, Bevacizumab (Avastin®), Genentech/Roche) is an angiogenesis inhibitor, a drug that slows the growth of new blood vessels. It is licensed to treat various cancers, including colorectal, lung, breast (outside the USA), glioblastoma (USA and Japan), kidney and ovarian. Bevacizumab is a humanized monoclonal antibody that inhibits vascular endothelial growth factor A (VEGF-A). VEGF-A is a chemical signal that stimulates angiogenesis in a variety of diseases, especially in cancer. Bevacizumab was the first clinically available angiogenesis inhibitor in the United States.

It is further contemplated that the therapies described above may be implemented in combination with all types of surgery. Approximately 60% of persons with cancer will undergo surgery of some type, which includes preventative, diagnostic or staging, curative and palliative surgery. These types of surgery may be used in conjunction with other therapies, such as oncolytic adenovirus therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and microscopically controlled surgery (Mohs surgery). It is further contemplated that the present invention may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection, systemic administration, or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well. Furthermore, in treatments involving more than a single treatment type (*i.e.,* construct, anticancer agent and surgery), the time between such treatment types may be about 1, 2, 3, 4, 5, 6, 7, 8, 9, 10, 11, 12, 13, 14, 15, 16, 17, 18, 19, 20, 21, 22, 23, or about 24 hours apart; about 1, 2, 3, 4, 5, 6, or 7 days apart; about 1, 2, 3, 4, or 5 weeks apart; and about 1, 2, 3, 4, 5, 6, 7, 8, 9, 10, 11, or 12 months apart, or more.

It also should be pointed out that any of the foregoing therapies may prove useful by themselves. In this regard, reference to chemotherapeutics and non- mutant oncolytic virus therapy in combination also should be read as contemplating that these approaches may be employed separately.

### IV. SCREENING METHODS

With adenovirus Δ24 and other mutant adenovirus that are unable to bind Rb, it is necessary for the Rb pathway to be defective in order for the cell to transcribe and translate viral proteins. The Rb pathway is required to be defective in the sense that it is not able to repress the transcription-activating activity of E2F. E2F activates the transcription of cellular genes and adenoviral DNA if its activity is not repressed. Examples of ways in which E2F could escape repression include, but are not limited to, Rb not being able to bind E2F (*i.e.,* E1A binding to Rb), overexpression of E2F, less Rb than E2F and situations in which Rb remains phosphorylated.

In addition, the present inventors have observed that the identification of a Th1 polarized immune response in subjects is predictive of successful treatment with the oncolytic adenoviruses of the present invention. Also, the presence of Th2 response may be an indicator of non-response. A particular Th1 marker is IL-12p70. Also, high levels of antibodies again tumor associated antigens such as NLRP4 maybe assessed and if found predicts response to the oncolytic viral therapy.

Th1 markers include IL-1β, IL-2, IL-8, IL-12, IL-18, IFN-γ, TNF-α, TNF-β, GMCSF, cleaved caspase 3, neopterin and β2-microglobuin. Th1 surface markers include CXCR3, CCR5, CCR1 and IL-12 recepter β1 and α chains. Th2 markers include IL-4, IL-5, IL-6, IL-10, IL-13, TGFβ and phosphorylated STAT3. Th2 surface markers include CXCR4, CCR3, CCR4, CCR7, CCR8, IL-1 receptor and CD30. Tumor associated antigens include BRAF, CABYR, CRISP3, CSAG3, CTAG2, DHFR, FTHL17, GAGE1, LDHC, MAGEA1, MAGEA3, MAGEA4, MAGEB6, MAPK1, MICA, MUC1, NLPR4, NYES01, P53, PBK, PRAME, SOX2, SPANXA1, SSX2, SSX4, SSX5, TSGA10, TSSK6, TULP2, XAGE2 and ZNF165. In particular, 1, 2, 3, 4, 5, 6, 7 or all 8 of CABYR, MAGEA1, MAGEA3, MAGEB6, NLPR4, NYES01, PBK, and ZNF165 are examined.

Antibodies can be used to detect adenoviral proteins (*e.g*., E1A), Rb, and other proteins of the Rb pathway, Th1 response, Th2 response or tumor associated antigens. In certain aspects of the invention, one or more antibodies may be produced that are immunoreactive with multiple antigens. These antibodies may be used in various diagnostic or therapeutic applications, described herein below.

As used herein, the term "antibody" is intended to refer broadly to any immunologic binding agent such as IgG, IgM, IgA, IgD and IgE. Generally, IgG and/or IgM are preferred because they are the most common antibodies in the physiological situation and because they are most easily made in a laboratory setting. Means for preparing and characterizing antibodies are also well known in the art (see, e.g., Harlow and Lane (1988).

Certain embodiments of the invention provide antibodies to antigens and translated proteins, polypeptides and peptides that are linked to at least one agent to form an antibody conjugate. In order to increase the efficacy of antibody molecules as diagnostic or therapeutic agents, it is conventional to link or covalently bind or complex at least one desired molecule or moiety. A reporter molecule is defined as any moiety which may be detected using an assay. Non-limiting examples of reporter molecules which have been conjugated to antibodies include enzymes, radiolabels, haptens, fluorescent labels, phosphorescent molecules, chemiluminescent molecules, chromophores, luminescent molecules, photoaffinity molecules, colored particles or ligands, such as biotin.

Certain examples of antibody conjugates are those conjugates in which the antibody is linked to a detectable label. "Detectable labels" are compounds and/or elements that can be detected due to their specific functional properties, and/or chemical characteristics, the use of which allows the antibody to which they are attached to be detected, and/or further quantified if desired.

Rb expression or adenoviral gene expression in a population of cells can be determined by western blot analysis using antibodies as probes to adenoviral proteins. The level of viral proteins detected would indicate whether viral protein expression is occurring in the cell.

Immunodetection methods for detecting biological components such as protein(s), polypeptide(s) or peptide(s) involved in adenoviral replication or the cellular Rb or p53 pathways may be employed. Some immunodetection methods include enzyme linked immunosorbent assay (ELISA), radioimmunoassay (RIA), immunoradiometric assay, fluoroimmunoassay, chemiluminescent assay, bioluminescent assay, and Western blot to mention a few. The steps of various useful immunodetection methods have been described in the scientific literature, such as, e.g., Doolittle and Ben-Zeev (1999); Gulbis and Galand (1993); De Jager *et al.* (1993); Nakamura *et al.* (1987).

In terms of antigen detection, the biological sample analyzed may be any sample that is suspected of containing an antigen, such as, for example, a tissue section or specimen, a homogenized tissue extract, a cell, an organelle, separated and/or purified forms of any of the above antigen-containing compositions, or even any biological fluid that comes into contact with the cell or tissue, including blood and/or serum, although tissue samples or extracts are preferred.

In general, the detection of immunocomplex formation is well known in the art and may be achieved through the application of numerous approaches. These methods are generally based upon the detection of a label or marker, such as any of those radioactive, fluorescent, biological and enzymatic tags. U.S. Patents concerning the use of such labels include 3,817,837; 3,850,752; 3,939,350; 3,996,345; 4,277,437; 4,275,149 and 4,366,241). Of course, one may find additional advantages through the use of a secondary binding ligand such as a second antibody and/or a biotin/avidin ligand binding arrangement, as is known in the art.

A tumor may be biopsied and the above tests performed upon it to determine the presence or absence of glioma cells, either prior to, during or aftern treatment. An example of a biopsy protocol is as follows. The stereotactic biopsy is the precise introduction of a metal probe into the brain tumor, cutting a small piece of the brain tumor, and removing it so that it can be examined under the microscope. The patient is transported to the MRI or CAT scan suite, and the frame is attached to the scalp under local anesthesia. The "pins" of the frame attach to the outer table of the skull for rigid fixation (frame will not and can not move from that point forward until completion of the biopsy). The scan (MRI or CT) is obtained. The neurosurgeon examines the scan and determines the safest trajectory or path to the target. This means avoiding critical structures. The spatial co-ordinates of the target are determined, and the optimal path is elected. The biopsy is carried out under general anesthesia. A small incision is created over the entry point, and a small hole is drilled through the skull. The "dura" is perforated, and the biopsy probe is introduced slowly to the target. The biopsy specimen is withdrawn and placed in preservative fluid for examination under the microscope. Often the pathologist is present in the biopsy suite so that a rapid determination of the success of the biopsy can be made.

### V. EXAMPLES

The following examples are included to demonstrate preferred embodiments of the invention. It should be appreciated by those skilled in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result. More specifically, it will be apparent that certain agents that are both chemically and physiologically related may be substituted for the agents described herein while the same or similar results would be achieved.

### EXAMPLE 1 - METHODS

A Phase 1, dose-escalating, two-part study of DNX-2401 for high-grade glioma was initiated under an investigator-sponsored IND at MD Anderson Cancer Center in Houston Texas. To be eligible for the study, patients were required to have histologically-proven, recurrent high-grade malignant glioma. Group A of the study evaluated the direct intratumoral injection of a single dose of DNX-2401 into a growing area of biopsy-confirmed recurrent glioma, while Group B evaluated the injection of a divided dose of virus into the resection bed following glioma excision. The starting dose for both study groups was 10⁷ (*e.g.,* 1 x 10⁷) viral particles (vp), with a plan to dose escalate in half-log increments up to 3¹⁰ vp. The primary objectives of the study were to determine the safety, tolerability, feasibility, and biological effect of injecting DNX-2401 into human brain tumors *in situ.*

Patients in Group A received direct intratumoral injection through a needle and underwent standard dose escalation by cohort. Tumors may or may not have been surgically resectable. The assigned dose levels were: 1 × 10⁷, 3 × 10⁷, 1 × 10⁸, 3 × 10⁸, 1 × 10⁹, 3 × 10⁹, 1 × 10¹⁰, and 3 × 10¹⁰ viral particles (vp). Patients were observed for 28 days following virus injection before patients in the next cohort were enrolled and treated.

Group B only included patients with resectable tumors. Patients in Group B received direct intratumoral injection through a permanently implanted catheter in the center of the tumor and then underwent standard dose escalation by cohort (*i.e.,* dose levels 1 × 10⁷, 3 × 10⁷, 1 × 10⁸, 3 × 10⁸, 1 × 10⁹, 3 × 10⁹, 1 × 10¹⁰, and 3 × 10¹⁰ viral particles (vp)). Dose escalation for group B was similar to group A, except that group B lagged behind group A by one dose level. Patients in Group A were not included in Group B. After 14 days of observation, the tumor was resected en bloc fashion with the catheter in place to provide biological specimens for pathological and molecular analyses. After tumor removal, additional DNX-2401 was injected into the residual tumor surrounding the resection cavity (*i.e*., intramural injection into the tumor bed).

Group A completed the enrollment of 25 subjects in September 2012. The maximum dose achieved was 3 × 10¹⁰ vp as planned. Enrollment into group B that evaluated DNX-2401 as an adjunct to surgery was initiated later and enrolled 12 subjects with the maximum dose of 3 × 10⁸ vp. Follow-up was scheduled to occur at monthly intervals for 4 months, every 2 months for 2 years, and every 4 months for life thereafter for both treatment groups. Patients were and will be monitored for toxicity and symptoms, and evaluated using magnetic resonance imaging (MRI), spinal tap, and other tests as appropriate based on clinical standards of care for the duration of the study.

### EXAMPLE 2 - RESULTS

Study assessments for both treatment groups were performed at regular time intervals as outlined in the schedule of assessments. Data were recorded in electronic case report forms per MD Anderson standards and intra-institutionally monitored through MD Anderson's IND office approximately every 4 weeks. The data presented are unaudited and should be considered preliminary at this time.

**Extent of Exposure.** The maximum virus exposure for a patient in group A consisted of 3 X 10¹⁰ vp following intratumoral delivery (4 patients). A maximum dose of 6 x 10⁸ vp was delivered to three patients in group B.
**Table 1 - Exposure**

| | **Number Patients** | **Total Dose (vp)** | **Comments** |
|---|---|---|---|
| **Group A (N=25)** | | | |
| Cohort 1 - 1 X 10⁷ | 3 | 1 X 10⁷ | Intratumoral |
| Cohort 2 - 3 X 10⁷ | 3 | 3 X 10⁷ | Intratumoral |
| Cohort 3 - 1 X 10⁸ | 3 | 1 X 10⁸ | Intratumoral |
| Cohort 4 - 3 X 10⁸ | 3 | 3 X 10⁸ | Intratumoral |
| Cohort 5 - 1 X 10⁹ | 3 | 1 X 10⁹ | Intratumoral |
| Cohort 6 - 3 X 10⁹ | 3 | 3 X 10⁹ | Intratumoral |
| Cohort 7 - 1 X 10¹⁰ | 3 | 1 X 10¹⁰ | Intratumoral |
| Cohort 8 - 3 X 10¹⁰ | 4 | 3 X 10¹⁰ | Intratumoral |

| **Group B (N=12)** | | | |
|---|---|---|---|
| Cohort 1 - 1 X 10⁷ | 3 | 2 X 10⁷ | Intratumoral/Intramural |
| Cohort 2 - 3 X 10⁷ | 3 | 6 X 10⁷ | Intratumoral/Intramural |
| Cohort 3 - 1 X 10⁸ | 3 | 2 X 10⁸ | Intratumoral/Intramural |
| Cohort 4 - 3 X 10⁸ | 3 | 6 X 10⁸ | Intratumoral/Intramural |

**Table 2 - Patient Disposition**

| **Overall** | |
|---|---|
| Number screened | 48 |
| Number screen failed | 11 |

| **Group A - intratumoral administration** | |
|---|---|
| Number treated | 25 |
| Currently on-study in follow-up | 3 |

| **Group B - intratumoral/intramural administration** | |
|---|---|
| Number treated | 12 |
| Currently on-study in follow-up | 2 |

A total of 37 patients were enrolled, with 25 patients treated in group A (intratumoral administration of DNX-2401) and 12 patients treated in group B (intratumoral/intramural administration of DNX-2401). As of March 2013, two of 25 patients treated in group A and one of 12 patients treated in group B remain on the study and are being followed per protocol.

Of the 37 enrolled, 29 patients had histologically-confirmed glioblastoma, seven had anaplastic astrocytoma, and one patient had gliosarcoma. Upon study entry, 27 patients had experienced a first recurrence and for 10 patients tumor recurrence had occurred twice. In terms of functional impairment, a Karnofsky score of 90-100 was reported for 28 patients (20 patients in group A and eight patients in group B) and a Karnofsky score of 70-80 was reported for nine patients (five patients in group A and four patients in group B).

**Group A (25 enrolled).** All patients who had measurable tumor and completed a single-dose treatment were considered evaluable for response (N=25). Patients with histopathologically-confirmed recurrent high-grade glioma were heavily pre-treated for the disease at the time of study enrollment. All patients had received radiotherapy with concomitant temozolomide.

All patients (who may or may not have been surgically accessible) completed treatment successfully (N=25) up to a dose of 3 × 10¹⁰ vp. Although this was a dose-escalation study spanning four orders of magnitude, all patients were included in the efficacy analysis. A complete response (CR) was observed in 4 (16%) patients, partial response (PR) in 2 (8%), stable disease (SD) in 7 (28%) and progressive disease (PD) in 12 (48%). Clinical benefit (CR+PR+SD) was seen in 13 (52%) of patients. The lowest dose at which a response (CR) was observed by RANO criteria was at 1 x 10⁸ vp (cohort 3). This patient went on to be declared a complete response and is alive and on study at 38 months post-DNX-2401 treatment. The second CR was in cohort 7 at a dose of 1e10 vp.

All patients were included in a PFS (progression free survival) and OS (overall survival) analysis. A total of 7 (28%) patients achieved at least 6 months progression-free survival (PFS-6). Median OS for all subjects was 8 months and 1 year. OS was 32% with 1 patient alive (5.5 months) who has not yet achieved the one-year survival mark. Median OS for responders (CR+PR) was 14 months. Six patents (24%, 2 CR, 1 PR, 3SD) remain alive as of March 2013, 5 of whom have survived more than one year from treatment.

**Group B (12 enrolled).** Patients with histopathologically-confirmed recurrent high-grade glioma were heavily pre-treated by the time of enrollment. All patients had received radiotherapy with concomitant temozolomide.

All patients completed treatment successfully (N=12) up to a dose of 3 × 10⁸ vp (for a total exposure of 6 × 10⁸ vp fractionally delivered on days 0 and 14). Three patients (25%) had measurable disease following resection 14 days post intratumoral injection, and 9 (75%) patients had no measurable disease as a result of surgery. Of those 3 patients with measurable disease, a partial response (PR) was achieved for 1 and stable disease (SD) for 2 patients. Of the 9 patients (75%) with no measurable disease, 5 (56%) patients exhibited stable disease (SD), which was determined by the absence of recurrence. Clinical benefit (CR+PR+SD) for all patients in the B arm was 66%. Overall, at least 3 (25%) patients were progression-free at 6 months. Seven (58%) of patients remain alive as of March, 2013.

**Mechanism of tumor response.** Key advantages of single administration of DNX-2401 as monotherapy for recurrent high-grade glioma include:
- Persistent anti-tumor response with characteristic changes on MRI
- Minimal if any toxic side effects thereby enhancing patient quality of life
- Does not preclude the use of other anti-cancer agents or treatments in combination
- Potential for complete anti-tumor response

Tumor response to therapy appears to be accompanied by signature changes on contrast MRI. These include early, global changes in contrast pattern ("bunch of grapes") followed in some instances by the emerge of a "thread" pattern or what resembles "soap bubbles." By several months post DNX-2401 treatment, several tumors appear to progress and have less defined borders. This is now thought to be caused by inflammation, such as that seen with other immunotherapy products. This will then change to the more distinct, smaller tumor, which in some instances goes on to a complete response.

The evidence that the characteristic changes on MRI observed during this trial are related to response is derived, in part, from pathology reports on surgically resected tumors. Two tumors were resected several months after DNX-2401 therapy in response to what appeared to be tumor progression. In both instances, pathologists reported that the tumors were >80% destroyed ("treatment related necrosis") with the remaining tumor infiltrated by an admix of immune cells (subsequently shown to be predominantly CD8 T cells). This suggests to us that infection by DNX-2401 may be triggering an effective antitumor immune response or otherwise destabilizing the tumor. If this finding is confirmed, it could account for the persistence of the anti-glioma effects observed (by MRI scans and/or post-treatment resection) in the tumors of several subjects following a single DNX-2401 injection.

Anti-tumor response to treatment is an especially important endpoint in this disease, as displacement of normal brain tissue due to rapidly growing tumor eventually results in severe disability and death. Overall, there is a high unmet need for a new modality of attack on gliobastoma that, with minimal morbidity, can positively impact the course of disease. As a new agent associated with fewer adverse effects while decreasing the risk of drug resistance and off-target toxicity, DNX-2401 has the potential to be safer and more effective than current therapies for recurrent gliobastoma. Moreover, it appears to exceed even the efficacy of Avastin®, which achieved an objective response rate of 25.9% (22/85). Twenty-two patients achieved partial remission, with median duration of response 4.2 months.

**Biomarkers and Anti-tumor Immunity.** The experience gained during the Phase I clinical trial has revealed an interesting correlation between patients that responded to the DNX-2401 oncolytic therapy and those that did not. This correlation was based on serum assays for the antibodies to cancer-related antigens (CRA). Because CRA antigens are not present in normal tissue and are "turned on" by cancers as they progress, they are informative about the nature of disease within cancer.

The inventors tested patients entering the Phase I clinical trial for the presence or absence of antibodies to 31 distinct CRA's. They specifically looked for patients that expressed antibodies for these antigens prior to receiving DNX-2401 and also for patients that developed antibodies post-treatment. Surprisingly, patients with tumors that had a radiographic response to DNX-2401 had low or no humoral antibody response to the defined set of tumor antigens

The lack of an antibody response suggested that a response to DNX-2401 therapy may be based on a cellular versus humoral immune response. Because of this, the inventors looked at the cytokine profile of responders *versus* non-responders with the expectation that strong responders would exhibit more of a Th1 (cytotoxic T8 cell) polarization and non-responders would show a profile more consistent with a Th2 (antibody-producing) profile. In general, the Th1 response is characterized by an increase in antigen-specific interferon-gamma (IFN-γ), IL-12, and complement-fixing antibodies, whereas the Th2 phenotype is characterized by production of IL-4, IL-5, IL-10, and an increase in IgE, IgA, and overall IgG antibodies. This expectation was confirmed by cytokine expression (using ELISA semiquantitative MSD assays).

The measurement of antibodies in patient sera has several advantages when compared to other more conventional biomarker classes. First, serum is a readily accessible tissue requiring relatively non-invasive sampling. Second, antibodies provide an amplified response and their relative abundance enables early warning or detection of small changes. Third, a tissue biopsy is not required, which is both invasive, unpleasant to the patient and depending on the tumor accessibility, often contains a mixture of various cell types.

**Safety.** To date, there have been no unexpected toxicities associated with the administration of DNX-2401 for brain tumors. Adverse events have generally been mild to moderate in severity and unrelated to virus following both types of administration (*i.e.,* intratumoral and intramural). No patients discontinued the study because of an adverse event, and the analysis of patient sera, saliva and urine has not demonstrated virus shedding. All SAEs of death were considered unrelated to DNX-2401. This safety profile is significant in that Avastin® can cause severe toxicity in patients. None of these patients experienced any adverse events related to the drug, and thus there are absolutely no safety or toxicity concerns with DNX-2401 at present.

Safety data from two additional clinical studies conducted with DNX-2401 support the safety observced in the brain clinical study. 21 women with gynelogic malignancies received daily x3 days at doses ranging from le9 vp to 1e12 vp/day in a gynecological cancer (Kimball et al., 2010; ClinicalTrials.gov Identifier: NCT00562003). In addition, 12 patients with high grade glioma were treated and the investigators have reported that virus infusion is feasible and safe in tumor and surrounding brain. Adverse events were temporary and serious adverse events have been unrelated to the virus (ClinicalTrials.gov Identifier: NCT01582516).

Many of the current cancer therapeutics are limited in their use due to the severe toxicity to the patients. For example, >99% of the patients patients enrolled in AVF3708g who received bevacizumab reported to experience adverse events including fatigue, headaches, hypertension, bleeding/hemorrhage, venous/arterial thromboembolic events. Other serious events included wound-healing complications, proteinuria, gastrointenstinal perforation. Based on the historical data of toxicity of cancer therapeutics, no advers events observed with DNX-2401 is unexpected and surprising.

**Examples of specific patient outcomes.** Three patients, who have only received glioblastoma treatment with DNX-2401, have continued with follow up. Details are provided below.

Patient #12. Patient #12 (56 year-old white female) was diagnosed that led to tumor resection and subsequent treatment with chemotherapy that consisted of temozolomide and dasatinib, and radiotherapy. Sshe was enrolled in Group A (intratumoral administration of DNX-2401) and randomized to Cohort 3. She received 4 intratumoral injections/1 mL of DNX-2401 at a total dose of 1 × 10⁸ viral particles (vp). Clinically, the patient has done very well. All neurologic symptoms subsequently resolved over the first 6 months. She did have a slight increase in the overall size of the brain lesion that had the appearance of pseudo-progression/inflammation; however, this was followed by continuous tumor shrinkage. During the study, she had not experienced a serious adverse event (SAE). All AEs have been considered unrelated to DNX-2401 with the excention of lymphocytopenia that was considered unlikely related and secondary to temozolomide. At 32 months, the patient is currently alive and being followed for survival. The last MRI only revealed scarring and contraction of surrounding brain that was considered a complete response to virus treatment alone. The patient is continuing to do well and is being monitored every 4-months. She is feeling well and reports walking 60 minutes per day and gardening, activities that she was unable to undertake prior to receiving DNX-2401.

Patient #33. Patient #33 (40 year-old white female) underwent primary tumor resection identifying gliobastoma. She received temozolomide chemotherapy and radiotherapy. She was enrolled in Group A (intratumoral administration of DNX-2401) and randomized to Cohort 7. She received 4 intratumoral injections/mL of DNX-2401 at a total dose of 1 × 10¹⁰ vp. She did well during the month immediately following injection, with neurological symptoms resolving especially expressive aphasia and partial complex seizures. Additionally, the contrast-enhancing mass as well as the FLAIR abnormality on serial MR scans had virtually disappeared. The patient has not experienced a serious adverse event during the study. Overall, the patient appears to have had a complete response by McDonald criteria. Since DNX-2401 administration, the patient is currently alive and doing well. To date, she is neurologically symptom-free 16 months post injection of DNX-2401.

Patient #42. Patient #42 (white female) underwent primary tumor resection that confirmed glioblastoma. She received radiotherapy and also received 4 courses of chemotherapy as follows: temozolomide, memantine temozolomide again, and macitentan. She was enrolled in Group B (intratumoral/intramural administration of DNX-2401) and randomized to Cohort 4. She received one intratumoral injection/mL of DNX-2401 at a total dose of 3 × 10⁸ vp, followed by 10 intramural injections/1 mL of DNX-2401 at a total dose of 3 × 10⁸ vp. Following resection, there was no measurable disease. During study participation she has not experienced a serious adverse event. Patient is currently alive and doing well.

One of skill in the art readily appreciates that the present invention is well adapted to carry out the objectives and obtain the ends and advantages mentioned as well as those inherent therein. Methods, procedures and techniques described herein are presently representative of the preferred embodiments and are intended to be exemplary and are not intended as limitations of the scope.

### VI. References

U.S. Patent 3,817,837
U.S. Patent 3,850,752
U.S. Patent 3,939,350
U.S. Patent 3,996,345
U.S. Patent 4,275,149
U.S. Patent 4,277,437
U.S. Patent 4,366,241
U.S. Patent 8,168,168
U.S. Patent 6,824,771
U.S. Patent Appln. 20030138405
Andreanski et al., Cancer Res., 57:1502-1509, 1997.
Chase et al., Nat. Biotechnol., 16, 444-448, 1998.
Chintala et al., Oncogene, 15(17):2049-2057, 1997.
Coffey et al., Science, 282:1332-1334, 1998.
Collins and James, FASEB J., 7:926-930, 1993.
Costello et al., Cancer Res., 56:2405-2410, 1996.
Costello et al., Cancer Res., 57:1250-1254, 1997.
De Jager et al., Semin. Nucl. Med., 23(2):165-179, 1993.
Doolittle and Ben-Zeev, Methods Mol Biol, 109:215-237, 1999.
Dyson and Harlow, Cancer Surv., 12:161-195, 1992.
Flint and Shenk, Annu. Rev. Genet., 31:177-212, 1997.
Freytag et al., Hum. Gene Ther., 9:1323-1333, 1998.
Fueyo et al., Archives of Neurology, 56(4):445-448, 1999.
Fueyo et al., J. Natl. Cancer Inst., 95:652-60, 2003.
Fueyo et al., Nat. Med., 4:685-690, 1998b.
Fueyo et al., Nature Medicine, 4(6):685-690, 1998.
Fueyo et al., Neurology, 50:1307-1315, 1998c.
Fueyo et al., Neurology, 51:1250-1255, 1998a.
Fueyo et al., Oncogene, 12:103-110, 1996a.
Fueyo et al., Oncogene, 13:1615-1619, 1996b.
Fueyo et al., Oncogene, 19:2-12, 2000.
Furnari et al., Cancer Surv., 25, 233-275, 1995.
Geoerger et al., Cancer Res., 62(3):764-772, 2002.
Gomez-Manzano et al., In: Gene Transfer and Therapy for Neurological Disorders, Chiocca and Breakefield (Eds.), Human Press Inc.: NJ, 201-225, 1998.
Grunhaus and Horwitz, Seminar in Virology, 3:237-252, 1992.
Gulbis and Galand, Hum. Pathol., 24(12):1271-1285, 1993.
Hamel et al., J. Neurooncol., 16:159-165, 1993.
Harlow and Lane, In: Antibodies: A Laboratory Manual, Cold Spring Harbor Laboratory, Cold Spring Harbor, NY, 346-348, 1988.
Heise et al., Nat. Med., 3:639-645, 1997.
Henson et al., Ann. Neurol., 36:714-721, 1994.
Hess et al., Neuro-Oncol., 1(4):282-288, 1999.
Hirvonen et al., Br. J. Cancer, 69:16-25, 1994.
Jacotot, Acad. Sci. Hebd. Seances Acad. Sci., 264(22):2602-2603, 1967.
Jen et al., Cancer Res., 54:6353-6358, 1994.
Kaelin, Bioessays, 21(11):950-8, 1999.
Karlsson et al., EMBO J., 5:2377-2385, 1986.
Kimball et al., Clin Cancer Research, 16(21); 5277-87, 2010.
Kirn et al., Nat. Med., 4, 1341-1342, 1998.
Kirn, Expert Opin. Biol. Ther., 1(3):525-538, 2001.
Kruse et al., J. Neuro-Oncol., 19:161-168, 1994.
Kyritsis and Yung, Baillieres Clinical Neurology, 5(2):295-305, 1996.
Kyritsis et al., Mol. Carcinog., 15:1-4, 1996b.
Kyritsis et al., Oncogene, 12:63-67, 1996a.
Martuza et al., Science, 10:854-856, 1991.
MacDonald et al., J. Clin. Oncol., 8(7):1277-80, 1990.
Mineta et al., Nat. Med., 9:938-943, 1995.
Nakamura et al., In: Handbook of Experimental Immunology (4th Ed.), Weir et al. (Eds), 1:27, Blackwell Scientific Publ., Oxford, 1987.
Nevins, Science, 258:424-429, 1992.
PCT Appln. WO 95/27071
PCT Appln. WO 96/12406
Puumalainen et al., Hum. Gene Ther., 9:1769-1774, 1998.
Remington's Pharmaceutical Sciences, 15th ed., 33:624-652, Mack Publishing Company, Easton, PA, 1980.
Rouslahti and Rajotte, Annu. Rev. Immunol., 18, 813-827, 2000.
Schiffer, Forum, 8(3):244-255, 1998.
Schmidt et al., Cancer Res., 54:6321-6324, 1994.
Shapiro and Shapiro, Oncology, 12(2):233-240, 1998.
Sidransky et al., Nature, 355:846-847, 1992.
Suzuki et al., Clin. Cancer Res., 7:120-126, 2001.
Ueki et al., Cancer Res., 56:150-153, 1996.
Vile, Cancer Gene Ther., 9(12):1062-1067, 2002.
Whyte et al., Cell, 56:67-75, 1989.
Whyte et al., Cell, 62:257-265, 1988.
Wildner et al., Cancer Res., 59:410-413, 1999.
Yan et al., J. Virol., 77(4):2640-2650, 2003.

## Claims

1. An oncolytic adenovirus for use in one or more of:
(i) a method for achieving greater than 25% reduction in tumor burden when treating a glioma in a human patient without producing of an adverse event resulting from said oncolytic adenovirus that is sufficient to cause termination of said treatment;
(ii) a method for achieving a six-month progression-free survival when treating a glioma in a human patient without producing of an adverse event resulting from said oncolytic adenovirus that is sufficient to cause termination of said treatment; and
(iii) a method for achieving tumor necrosis when treating a glioma in a human patient without producing of an adverse event resulting from said oncolytic adenovirus that is sufficient to cause termination of said treatment,
wherein the method comprises contacting the glioma, in the human patient identified as having the glioma, with the oncolytic adenovirus, and wherein the oncolytic adenovirus is an oncolytic adenovirus with E1A polypeptide that cannot bind Rb, and comprises a fiber protein with an RGD amino acid inserted in the HI domain.

2. The oncolytic adenovirus for use in a method of claim 1, wherein the oncolytic adenovirus is a Δ24 adenovirus.

3. The oncolytic adenovirus for use in a method of claim 1, wherein the patient is identified as having a glioma by a method that comprises tumor imaging, and said method further comprises obtaining a biopsy of said tumor after imaging and before treatment with the oncolytic adenovirus.

4. The oncolytic adenovirus for use in a method of claim 1, wherein said glioma is:
(a) resectable, preferably wherein the method comprises resecting the glioma following the treatment with the oncolytic adenovirus, and more preferably wherein a post-resection tumor bed is treated with said oncolytic adenovirus, and yet more preferably wherein a post-resection catheter is implanted into said patient and said oncolytic adenovirus is delivered via said catheter;
(b) not resectable, and preferably wherein the method comprises resecting the glioma following the treatment with the oncolytic adenovirus.

5. The oncolytic adenovirus for use in a method of claim 1, wherein the glioma is contacted with the adenovirus by delivery of the adenovirus intracranially into the patient, for example wherein delivery comprises intratumoral injection and optionally wherein multiple intratumoral injections are performed.

6. The oncolytic adenovirus for use in a method of claim 1, wherein the glioma is an astrocytoma, an oligodendroglioma, an anaplastic glioma, a glioblastoma, an ependymoma, a meningioma, a pineal region tumor, a choroid plexus tumor, a neuroepithelial tumor, an embryonal tumor, a peripheral neuroblastic tumor, a tumor of cranial nerves, a tumor of the hemopoietic system, a germ cell tumors or a tumor of the sellar region, and preferably wherein the glioma is a glioblastoma.

7. The oncolytic adenovirus for use in a method of claim 1, wherein the oncolytic adenovirus is administered:
(a) via slow infusion over a period of minimum 10 minutes with a needle; or
(b) stereotactly into more than one site in a glioma in said patient.

8. The oncolytic adenovirus for use in a method of claim 1, wherein the method further comprises administering to the patient a second therapy, wherein the second therapy is anti-angiogenic therapy, chemotherapy, immunotherapy, surgery, radiotherapy, immunosuppresive agents, or gene therapy with a therapeutic polynucleotide, and optionally:
(a) wherein the second therapy is administered to the patient before administration of the composition comprising the oncolytic adenovirus;
(b) wherein the second therapy is administered to the patient at the same time as administration of the composition comprising the oncolytic adenovirus;
(c) wherein the second therapy is administered to the patient after administration of the composition comprising the oncolytic adenovirus; or
(d) wherein the chemotherapy comprises an alkylating agent, mitotic inhibitor, antibiotic, or antimetabolite.

9. The oncolytic adenovirus for use in a method of claim 1, wherein from about 10³ to about 10¹⁵ viral particles are administered to the patient, optionally about 10⁵ to about 10¹² viral particles or about 10⁷ to about 10¹⁰ viral particles.

10. The oncolytic adenovirus for use in a method of claim 1, wherein said subject is further selected based on the presence of a Th1 response, and optionally wherein said Th1 response is **characterized by** an increase in antigen-specific interferon-gamma (IFN-γ), IL-12, and complement-fixing antibodies.

11. The oncolytic adenovirus for use in a method of claim 1, for use in 2 or more of methods i)-iii).

12. The oncolytic adenovirus for use in a method of claim 1, for use in all 3 of methods i)-iii).

13. The oncolytic adenovirus for use in a method of claim 1, wherein said glioma is recurrent.

14. The oncolytic adenovirus for use in a method of claim 1, wherein said glioma has failed one or more primary glioma therapies.

15. An oncolytic adenovirus for use in one or more of:
(i) a method for treating a glioma in a human patient population and achieving a clinical benefit in 30% of said patients, with clinical benefit defined by complete responders + partial responders plus stable disease;
(ii) a method for treating a glioma in a human patient population and achieving a 25% six-month progression-free survival; and
(ii) a method for treating a glioma in a human patient population and achieving a 12 month median survival for responders, with responders defined by complete responders + partial responders,
wherein the method comprises contacting the gliomas of a population of the human patients that have been identified as having glioma with the oncolytic adenovirus, and wherein the oncolytic adenovirus is an oncolytic adenovirus with E1A polypeptide that cannot bind Rb, and comprises a fiber protein with an RGD amino acid inserted in the HI domain.

## Patentansprüche

1. Onkolytisches Adenovirus zur Verwendung in:
(i) einem Verfahren zum Erreichen einer Verringerung der Tumorlast um mehr als 25 % bei der Behandlung eines Glioms bei einem menschlichen Patienten, ohne ein unerwünschtes Ereignis hervorzurufen, das aus dem onkolytischen Adenovirus resultiert und ausreicht, um die Behandlung zu beenden;
(ii) einem Verfahren zum Erreichen eines sechsmonatigen progressionsfreien Überlebens bei der Behandlung eines Glioms bei einem menschlichen Patienten, ohne ein unerwünschtes Ereignis hervorzurufen, das aus dem onkolytischen Adenovirus resultiert und ausreicht, um die Behandlung zu beenden; und/oder
(iii) einem Verfahren zum Erreichen einer Tumor-Nekrose bei der Behandlung eines Glioms bei einem menschlichen Patienten, ohne ein aus dem onkolytischen Adenovirus resultierendes unerwünschtes Ereignis zu erzeugen, das ausreicht, um die Behandlung zu beenden, wobei das Verfahren das Inkontaktbringen des Glioms bei einem menschlichen Patienten, bei dem das Gliom festgestellt wurde, mit dem onkolytischen Adenovirus umfasst und wobei das onkolytische Adenovirus ein onkolytisches Adenovirus mit E1A-Polypeptid ist, das Rb nicht binden kann, und ein Faserprotein mit einer in die H1-Domäne inserierten RGD-Aminosäure umfasst.

2. Onkolytisches Adenovirus zur Verwendung in einem Verfahren nach Anspruch 1, wobei das onkolytische Adenovirus ein Δ24-Adenovirus ist.

3. Onkolytisches Adenovirus zur Verwendung in einem Verfahren nach Anspruch 1, wobei der Patient durch ein Verfahren, das die Tumor-Bildgebung umfasst, als Patient mit einem Gliom identifiziert wird, und wobei das Verfahren ferner das Gewinnen einer Biopsie des Tumors nach der Bildgebung und vor der Behandlung mit dem onkolytischen Adenovirus umfasst.

4. Onkolytisches Adenovirus zur Verwendung in einem Verfahren nach Anspruch 1, wobei das Gliom:
(a) resezierbar ist, wobei vorzugsweise das Verfahren das Resezieren des Glioms nach der Behandlung mit dem onkolytischen Adenovirus umfasst und wobei stärker bevorzugt ein Tumorbett nach der Resektion mit dem onkolytischen Adenovirus behandelt wird und wobei noch stärker bevorzugt ein Katheter nach der Resektion in den Patienten implantiert wird und das onkolytische Adenovirus über den Katheter abgegeben wird;
(b) nicht resezierbar ist, und wobei vorzugsweise das Verfahren das Resezieren des Glioms nach der Behandlung mit dem onkolytischen Adenovirus umfasst.

5. Onkolytisches Adenovirus zur Verwendung in einem Verfahren nach Anspruch 1, wobei das Gliom durch intrakranielle Verabreichung des Adenovirus an den Patienten mit dem Adenovirus in Kontakt gebracht wird, wobei die Verabreichung beispielsweise eine intratumorale Injektion umfasst und wobei optional mehrere intratumorale Injektionen durchgeführt werden.

6. Onkolytisches Adenovirus zur Verwendung in einem Verfahren nach Anspruch 1, wobei das Gliom ein Astrozytom, ein Oligodendrogliom, ein anaplastisches Gliom, ein Glioblastom, ein Ependymom, ein Meningiom, ein Tumor der Pinealregion, ein Plexus-Aderhaut-Tumor, ein Neuroepitheltumor, ein Embryonaltumor, ein peripherer neuroblastischer Tumor, ein Tumor der Hirnnerven, ein Tumor des hämopoetischen Systems, ein Keimzelltumor oder ein Tumor der Sellarregion ist und wobei das Gliom vorzugsweise ein Glioblastom ist.

7. Onkolytisches Adenovirus zur Verwendung in einem Verfahren nach Anspruch 1, wobei das onkolytische Adenovirus folgendermaßen verabreicht wird:
(a) durch eine langsame Infusion über einen Zeitraum von mindestens 10 Minuten mit einer Nadel; oder
(b) oder stereotaktisch an mehr als eine Stelle in einem Gliom bei dem Patienten.

8. Onkolytisches Adenovirus zur Verwendung in einem Verfahren nach Anspruch 1, wobei das Verfahren ferner das Verabreichen einer zweiten Therapie an den Patienten umfasst, wobei die zweite Therapie eine anti-angiogene Therapie, Chemotherapie, Immuntherapie, Operation, Strahlentherapie, Immunsuppressiva oder Gentherapie mit einem therapeutischen Polynukleotid ist, und optional:
(a) wobei die zweite Therapie vor der Verabreichung der Zusammensetzung, umfassend das onkolytische Adenovirus, an den Patienten verabreicht wird;
(b) wobei die zweite Therapie dem Patienten gleichzeitig mit der Verabreichung der Zusammensetzung, die das onkolytische Adenovirus umfasst, verabreicht wird;
(c) wobei die zweite Therapie dem Patienten nach Verabreichung der Zusammensetzung, die das onkolytische Adenovirus umfasst, verabreicht wird; oder
(d) wobei die Chemotherapie ein Alkylierungsmittel, einen Mitoseinhibitor, ein Antibiotikum oder einen Antimetaboliten umfasst.

9. Onkolytisches Adenovirus zur Verwendung in einem Verfahren nach Anspruch 1, wobei dem Patienten etwa 10³ bis etwa 10¹⁵ virale Partikel verabreicht werden, optional etwa 10⁵ bis etwa 10¹² virale Partikel oder etwa 10⁷ bis etwa 10¹⁰ virale Partikel.

10. Onkolytisches Adenovirus zur Verwendung in einem Verfahren nach Anspruch 1, wobei der Patient weiterhin auf der Grundlage des Vorhandenseins einer Th1-Antwort ausgewählt wird, und wobei die Th1-Antwort gegebenenfalls durch eine Zunahme von antigenspezifischem Interferon-gamma (IFN-γ), IL-12 und komplementfixierenden Antikörpern gekennzeichnet ist.

11. Onkolytisches Adenovirus zur Verwendung in einem Verfahren nach Anspruch 1, zur Verwendung in 2 oder mehreren der Verfahren i)-iii).

12. Onkolytisches Adenovirus zur Verwendung in einem Verfahren nach Anspruch 1, zur Verwendung in allen 3 Verfahren i)-iii).

13. Onkolytisches Adenovirus zur Verwendung in einem Verfahren nach Anspruch 1, wobei das Gliom rezidivierend ist.

14. Onkolytisches Adenovirus zur Verwendung in einem Verfahren nach Anspruch 1, wobei das Gliom auf eine oder mehrere primäre Gliom-Therapien nicht angesprochen hat.

15. Onkolytisches Adenovirus zur Verwendung in:
(i) einem Verfahren zur Behandlung eines Glioms bei einer menschlichen Patientenpopulation und zur Erzielung eines klinischen Nutzens bei 30 % dieser Patienten, wobei der klinische Nutzen definiert ist durch vollständige Responder + partielle Responder plus stabile Krankheit;
(ii) einem Verfahren zur Behandlung eines Glioms bei einer menschlichen Patientenpopulation und zur Erzielung eines progressionsfreien Überlebens von 25 % nach sechs Monaten; und/oder
(iii) einem Verfahren zum Behandeln eines Glioms in einer menschlichen Patientenpopulation und zum Erzielen einer medianen 12-Monats-Überlebenszeit für Responder, wobei Responder durch vollständige Responder + partielle Responder definiert sind, wobei das Verfahren das Inkontaktbringen der Gliome einer Population der menschlichen Patienten, bei denen ein Gliom festgestellt wurde, mit dem onkolytischen Adenovirus umfasst und wobei das onkolytische Adenovirus ein onkolytisches Adenovirus mit E1A-Polypeptid ist, das Rb nicht binden kann, und ein Faserprotein mit einer in die H1-Domäne inserierten RGD-Aminosäure umfasst.

## Revendications

1. Adénovirus oncolytique destiné à être utilisé dans l'un ou plusieurs :
(i) d'un procédé permettant d'obtenir une réduction de plus de 25 % de la charge tumorale lors du traitement d'un gliome chez un patient humain sans entraîner d'effet indésirable résultant dudit adénovirus oncolytique, lequel effet indésirable est suffisant pour provoquer l'arrêt dudit traitement ;
(ii) d'un procédé permettant d'obtenir une survie sans progression de la maladie à six mois lors du traitement d'un gliome chez un patient humain sans entraîner d'effet indésirable résultant dudit adénovirus oncolytique, lequel effet indésirable est suffisant pour provoquer l'arrêt dudit traitement ; et
(iii) d'un procédé permettant d'obtenir une nécrose tumorale lors du traitement d'un gliome chez un patient humain sans entraîner d'effet indésirable résultant dudit adénovirus oncolytique, lequel effet indésirable est suffisant pour provoquer l'arrêt dudit traitement,
dans lequel le procédé comprend la mise en contact du gliome, chez le patient humain identifié comme étant atteint du gliome, avec l'adénovirus oncolytique, et dans lequel l'adénovirus oncolytique est un adénovirus oncolytique comportant un polypeptide E1A qui ne peut pas se lier à Rb, et comprenant une protéine fibreuse comportant un acide aminé RGD inséré dans le domaine H1.

2. Adénovirus oncolytique destiné à être utilisé dans un procédé selon la revendication 1, l'adénovirus oncolytique étant un adénovirus Δ24.

3. Adénovirus oncolytique destiné à être utilisé dans un procédé selon la revendication 1, dans lequel le patient est identifié comme étant atteint d'un gliome par un procédé qui comprend l'imagerie tumorale, et ledit procédé comprend en outre la réalisation d'une biopsie de ladite tumeur après l'imagerie et avant le traitement avec l'adénovirus oncolytique.

4. Adénovirus oncolytique destiné à être utilisé dans un procédé selon la revendication 1, dans lequel ledit gliome est :
(a) résécable, le procédé comprenant de préférence la résection du gliome après le traitement avec l'adénovirus oncolytique, et un lit tumoral post-résection étant de manière davantage préférée traité avec ledit adénovirus oncolytique, et un cathéter post-résection étant de manière davantage préférée encore implanté dans ledit patient et ledit adénovirus oncolytique étant administré par l'intermédiaire dudit cathéter ;
(b) non résécable, et le procédé comprenant de préférence la résection du gliome après le traitement avec l'adénovirus oncolytique.

5. Adénovirus oncolytique destiné à être utilisé dans un procédé selon la revendication 1, dans lequel le gliome est mis en contact avec l'adénovirus par administration de l'adénovirus au patient par voie intracrânienne, l'administration comprenant par exemple une injection intratumorale et de multiples injections intratumorales étant éventuellement effectuées.

6. Adénovirus oncolytique destiné à être utilisé dans un procédé selon la revendication 1, dans lequel le gliome est un astrocytome, un oligodendrogliome, un gliome anaplasique, un glioblastome, un épendymome, un méningiome, une tumeur de la région pinéale, une tumeur du plexus choroïde, une tumeur neuroépithéliale, une tumeur embryonnaire, une tumeur neuroblastique périphérique, une tumeur des nerfs crâniens, une tumeur du système hémopoïétique, des tumeurs des cellules germinales ou une tumeur de la région sellaire, et dans lequel le gliome est de préférence un glioblastome.

7. Adénovirus oncolytique destiné à être utilisé dans un procédé selon la revendication 1, l'adénovirus oncolytique étant administré :
(a) par perfusion lente au moyen d'une aiguille pendant au moins 10 minutes ; ou
(b) stéréotactiquement dans plus d'un site dans un gliome chez ledit patient.

8. Adénovirus oncolytique destiné à être utilisé dans un procédé selon la revendication 1, dans lequel le procédé comprend en outre l'administration au patient d'une seconde thérapie, dans lequel la seconde thérapie est une thérapie anti-angiogénique, une chimiothérapie, une immunothérapie, une chirurgie, une radiothérapie, des agents immunosuppresseurs ou une thérapie génique avec un polynucléotide thérapeutique, et éventuellement :
(a) dans lequel la seconde thérapie est administrée au patient avant l'administration de la composition comprenant l'adénovirus oncolytique ;
(b) dans lequel la seconde thérapie est administrée au patient en même temps que l'administration de la composition comprenant l'adénovirus oncolytique ;
(c) dans lequel la seconde thérapie est administrée au patient après l'administration de la composition comprenant l'adénovirus oncolytique ; ou
(d) dans lequel la chimiothérapie comprend un agent alkylant, un inhibiteur mitotique, un antibiotique ou un antimétabolite.

9. Adénovirus oncolytique destiné à être utilisé dans un procédé selon la revendication 1, dans lequel environ 10³ à environ 10¹⁵ particules virales sont administrées au patient, éventuellement environ 10⁵ à environ 10¹² particules virales ou environ 10⁷ à environ 10¹⁰ particules virales.

10. Adénovirus oncolytique destiné à être utilisé dans un procédé selon la revendication 1, dans lequel ledit sujet est en outre sélectionné sur la base de la présence d'une réponse Th1, et éventuellement dans lequel ladite réponse Th1 est **caractérisée par** une augmentation de l'interféron-gamma spécifique de l'antigène (IFN-γ), de l'IL-12 et des anticorps fixant le complément.

11. Adénovirus oncolytique destiné à être utilisé dans un procédé selon la revendication 1, à être utilisé dans 2 ou plus des procédés i) à iii).

12. Adénovirus oncolytique destiné à être utilisé dans un procédé selon la revendication 1, à être utilisé dans les 3 procédés i) à iii).

13. Adénovirus oncolytique destiné à être utilisé dans un procédé selon la revendication 1, dans lequel ledit gliome est récurrent.

14. Adénovirus oncolytique destiné à être utilisé dans un procédé selon la revendication 1, dans lequel ledit gliome n'a pas été traité par une ou plusieurs thérapies primaires de gliome.

15. Adénovirus oncolytique destiné à être utilisé dans l'un ou plusieurs :
(i) d'un procédé permettant de traiter un gliome dans une population de patients humains, et d'obtenir un avantage clinique chez 30 % desdits patients, un avantage clinique étant défini par des patients présentant une réponse complète + des patients présentant une réponse partielle plus une maladie stable ;
(ii) d'un procédé permettant de traiter un gliome dans une population de patients humains, et d'obtenir une survie sans progression de la maladie à six mois de 25 % ; et
(iii) d'un procédé permettant de traiter un gliome dans une population de patients humains, et d'obtenir une survie moyenne à 12 mois pour les patients présentant une réponse, les patients présentant une réponse étant définis par des patients présentant une réponse complète + des patients présentant une réponse partielle,
le procédé comprenant la mise en contact des gliomes d'une population de patients humains qui ont été identifiés comme étant atteints d'un gliome avec l'adénovirus oncolytique, et l'adénovirus oncolytique étant un adénovirus oncolytique comportant un polypeptide E1A qui ne peut pas se lier à Rb, et comprenant une protéine fibreuse comprenant un acide aminé RGD inséré dans le domaine H1.


### SEQUENCE LISTING

<110> DNAtrix, Inc.
<120> TREATMENT OF BRAIN CANCER WITH ONCOLYTIC ADENOVIRUS
<130> DNAT.P0004WO
<140> Unknown
   <141> 2014-06-13
<150> US 61/836,230
   <151> 2013-06-18
<160> 1
<170> PatentIn version 3.5
<210> 1
   <211> 9
   <212> PRT
   <213> Artificial sequence
<220>
   <223> Synthetic peptide
<400> 1
   Cys Asp Cys Arg Gly Asp Cys Phe Cys
   1               5